# EUROPEAN PATENT APPLICATION

(11) **EP 4 349 353 A1**
(43) Date of publication of application: **10.04.2024**
(21) Application number: 22816443.0
(22) Date of filing: 31.05.2022
(51) Int. Cl.: A61K 35/747, A61K 9/00, A61P 37/00, A61P 3/00, A23L 33/135, A61K 8/99, A61Q 19/00

(54) **LACTOBACILLUS RHAMNOSUS-DERIVED VESICLE AND USES THEREOF**

(30) Priority: 03.06.2021 KR 20210072078; 06.12.2021 KR 20210173139
(71) Applicant: MD Healthcare Inc., Seoul 03923 (KR)
(72) Inventor: KIM, Yoon-Keun, Paju-Si, Gyeonggi-do 10908 (KR)
(74) Representative: Dehns
(86) International application number: PCT/KR2022/007768
(87) International publication number: WO 2022/255784

(57) **Abstract**

The present invention relates to a *Lactobacillus rhamnosus*-derived vesicle and uses thereof, and to a composition for preventing, alleviating, or treating immune diseases or cellular aging-related diseases, comprising a *Lactobacillus rhamnosus-*derived vesicle as an active ingredient. The present inventors have confirmed that a *Lactobacillus rhamnosus*-derived vesicle increases cellular homeostasis to suppress the occurrence of cellular aging-related diseases, and suppresses the occurrence of immune diseases by inhibiting immune hypersensitivity reactions in an immune disease model induced by viral causative factors. Accordingly, the *Lactobacillus rhamnosus*-derived vesicle according to the present invention may be effectively used in the development of a drug or health functional food for preventing, alleviating, or treating viral infectious diseases, allergic immune diseases, autoimmune diseases, inflammatory diseases, cellular aging-related diseases, etc.

## Description

### [Technical Field]

The present invention relates to a composition for preventing, alleviating, or treating immune diseases and cellular aging-related diseases, comprising a *Lactobacillus rhamnosus-derived* vesicle as an active ingredient.

This application claims priority to and the benefit of Korean Patent Application Nos. 10-2021-0072078 and 10-2021-0173139 filed in the Korean Intellectual Property Office on June 3, 2021 and December 6, 2021, respectively, and all the contents disclosed in the specification and drawings of the applications are incorporated in this application.

### [Background Art]

Entering the twenty-first century, the importance of acute infectious diseases, which were recognized as contagious diseases in the past, has decreased. On the other hand, the disease pattern has changed in that chronic diseases caused by immune dysfunction and cellular aging in major organs of our body have become major diseases that reduce the quality of life and determine the human lifespan. This change in disease pattern is closely related to changes in dietary pattern. In the past when nutrition was insufficient, acute infectious diseases caused by malnutrition were the main cause of death, but recently, as nutrition has become abundant, chronic diseases related to immune dysfunction caused by overnutrition and cellular aging are becoming major problems.

Due to overnutrition, as fat accumulates not only in adipose tissue, where fat is normally stored, but also in blood vessels, the heart, the liver, the kidneys, and muscles, lipotoxicity is caused, leading to inflammation and aging of cells. In other words, lipids and fatty acids in the blood increase due to carbohydrate or lipid metabolism disorders, and as fatty acids increase in cells, an inflammatory response due to fatty acids occurs, and the inflammatory response causes cellular aging and death, resulting in dysfunction in the cardiovascular system and organs such as the liver, kidneys, muscles, and brain. Diseases caused by these etiological causes include cardiovascular diseases such as angina pectoris, thromboembolism, myocardial infarction, cardiomyopathy, and stroke, renal diseases such as chronic nephropathy and renal failure, musculoskeletal diseases such as osteoarthritis, gout, and osteoporosis, and degenerative cerebral diseases such as Alzheimer's disease and Parkinson's disease.

Immunity is a cellular defense mechanism against biological, chemical, physical, and mental stress, and occurs through innate immunity and adaptive immunity. In particular, when an inappropriate immune response occurs upon repeated exposure to pathogenic factors, diseases occur as a result of a chronic inflammatory response, and representative examples include allergic diseases caused by immune hypersensitivity reactions due to environmental pathogenic factors and autoimmune diseases that occur as a result of hypersensitivity reactions due to internally generated pathogenic risk factors. In addition, it has recently been revealed that immune function is closely related to the metabolic function of cells. With regard to the etiology of immune dysfunction caused by overnutrition, intracytoplasmic metabolites such as fatty acids and uric acid act as a danger signal and are recognized by a nucleotide-binding oligomerization domain (NLRP), which is a pattern recognition receptor that is present in the cytoplasm. NLRPs are known to induce apoptosis by forming inflammasomes and at the same time, secrete inflammatory mediators to induce inflammatory cell infiltration.

Meanwhile, it is known that the number of microorganisms that coexist in the human body reaches 100 trillion, which is larger than that of human cells, and the number of genes of microorganisms is 100-fold larger than that of humans. A microbiota or microbiome refers to a microbial community including bacteria, archaea and eukarya present in a given habitat.

Bacteria that coexist in our bodies and bacteria that exist in the surrounding environment secrete nanometer-sized vesicles to exchange information such as genes, low molecular compounds, and proteins with other cells. The mucosa forms a physical defense membrane through which particles having a size of 200 nanometers (nm) or more cannot pass, so that bacteria coexisting in the mucosa cannot pass through the mucosa, but bacteria-derived vesicles have a size of 200 nanometers or less, and thus relatively freely pass through epithelial cells via the mucosa to be absorbed in our bodies. As described above, although bacteria-derived vesicles are secreted from bacteria, they differ from bacteria in terms of their constituents, absorption rate in the body, and risk of side effects, and therefore, the use of bacteria-derived vesicles is completely different from that of living cells or has a significant effect.

Locally secreted bacteria-derived vesicles are absorbed through the epithelial cells of the mucosa to induce a local inflammatory response, and vesicles that have passed through the epithelial cells are systemically absorbed through the lymphatic vessels to be distributed to respective organs, and regulate metabolic and immune functions in the organs to which the vesicles are distributed. For example, vesicles derived from pathogenic gram-negative bacteria, such as *Escherichia coli,* are pathogenic nanoparticles that cause local colitis or food poisoning, are absorbed into vascular endothelial cells when absorbed by blood vessels to promote a systemic inflammatory response and blood coagulation by inducing an inflammatory response, and are also absorbed into muscle cells where insulin acts to cause insulin resistance and metabolic disease such as diabetes mellitus and like. In contrast, vesicles derived from beneficial bacteria may regulate a disease by regulating immune and metabolic dysfunctions caused by pathogenic vesicles.

Bacteria of the genus *Lactobacillus* are known as representative beneficial bacteria that secrete lactic acid. Among them, *Lactobacillus rhamnosus* is a Gram-positive bacillus that grows well not only in an anaerobic environment but also in an aerobic environment, is a symbiotic bacterium in our bodies, and is known to be an important bacterium in a fermentation process. Vesicles derived from Gram-positive bacteria such as *Lactobacillus rhamnosus* contain peptidoglycan and lipoteichoic acid, which are bacterial cell wall components, in addition to bacteria-derived proteins, metabolites, and nucleic acids.

However, there is no case in which vesicles secreted from *Lactobacillus rhamnosus* have been used to prevent or treat diseases related to immune dysfunction and cellular aging.

### [Disclosure]

### [Technical Problem]

As a result of intensive research to solve the conventional problem described above, the present inventors have confirmed that *Lactobacillus rhamnosus-derived* vesicles have a therapeutic effect on immune diseases and cellular aging-related diseases, and thereby completed the present invention.

The present invention is directed to providing a pharmaceutical composition for preventing, alleviating or treating one or more selected from the group consisting of immune diseases and cellular aging-related diseases, comprising vesicles derived from *Lactobacillus rhamnosus* as an active ingredient.

However, a technical problem to be achieved by the present invention is not limited to the aforementioned problems, and the other problems that are not mentioned may be clearly understood by a person skilled in the art from the following description.

### [Technical Solution]

To achieve the object of the present invention as described above, the present inventors cultured *Lactobacillus rhamnosus* bacteria, isolated vesicles from the culture medium, and orally administered the isolated vesicles to mice with a cellular aging-related disease caused by a high-fat diet. As a result, the body weight gain was suppressed, and renal and pulmonary functions were improved, and it was confirmed that this action is related to the inhibition of Th17 and Th2 hypersensitivity reactions. In addition, it was confirmed that the vesicle of the present invention enhances the activation of AMP-activated kinase (AMPK), which is a key signal that induces cellular homeostasis through autophagy in a metabolic stress situation such as starvation and exercise where ATP production does not occur well. In addition, it was confirmed that when the vesicles were orally administered to an immune disease mouse model induced by poly(I:C), which is a viral pathogen, systemic inflammation responses caused by immune hypersensitivity reactions mediated by interferon-gamma and IL-6 were inhibited, and locally, pulmonary inflammation caused by immune hypersensitivity reactions mediated by TNF-α and IL-6 was significantly inhibited. In addition, it was confirmed that the immune hypersensitivity reactions due to the viral causative factors described above are controlled by the vesicle acting to inhibit the expression of an NF-xB signal and inducible NOS (iNOS), which is a downstream signal thereof.

Therefore, the present invention provides a pharmaceutical composition for preventing or treating one or more selected from the group consisting of immune diseases and cellular aging-related diseases, comprising vesicles derived from *Lactobacillus rhamnosus* as an active ingredient.

In one embodiment of the present invention, the immune disease may be one or more selected from the group consisting of viral infectious diseases, allergic immune diseases, and autoimmune diseases, but is not limited thereto.

In another embodiment of the present invention, the viral infectious disease may be one or more selected from the group consisting of viral pneumonia, viral hepatitis, viral carditis, viral enteritis, viral nephritis, viral thromboembolism, shingles, and Meniere's disease, but is not limited thereto.

In still another embodiment of the present invention, the autoimmune disease may be one or more selected from the group consisting of systemic lupus erythematosus, rheumatoid arthritis, Sjogren's syndrome, Behcet's disease, ankylosing spondylitis, and multiple sclerosis, but is not limited thereto.

In yet another embodiment of the present invention, the allergic immune disease may be one or more selected from the group consisting of atopic dermatitis, psoriasis, allergic conjunctivitis, allergic rhinitis, allergic asthma, hypersensitivity pneumonitis, food allergies, celiac disease, irritable bowel syndrome, and anaphylaxis, but it is not limited thereto.

In yet another embodiment of the present invention, the cellular aging-related disease may be one or more selected from the group consisting of one or more cardiovascular diseases selected from the group consisting of angina pectoris, myocardial infarction, cardiomyopathy, thromboembolism, and stroke; one or more hepato-biliary-pancreatic diseases selected from the group consisting of alcoholic steatohepatitis, cirrhosis, chronic cholangitis, chronic cholecystitis, and chronic pancreatitis; one or more musculoskeletal diseases selected from the group consisting of gout, osteoarthritis, and osteoporosis; and one or more neurological diseases selected from the group consisting of Alzheimer's disease, Parkinson's disease, and macular degeneration, but is not limited thereto.

As yet another exemplary embodiment of the present invention, the vesicles may have an average diameter of 10 to 1000 nm, but are not limited thereto.

In yet another embodiment of the present invention, the vesicles may be isolated from the culture medium of *Lactobacillus rhamnosus,* but are not limited thereto.

In yet another embodiment of the present invention, the vesicles may be isolated from food manufactured by adding *Lactobacillus rhamnosus,* but are not limited thereto.

As another exemplary embodiment of the present invention, the vesicles may be naturally secreted or artificially produced from *Lactobacillus rhamnosus,* but the present invention is not limited thereto.

In addition, the present invention provides a food composition for preventing or alleviating one or more selected from the group consisting of immune diseases and cellular aging-related diseases, comprising vesicles derived from *Lactobacillus rhamnosus* as an active ingredient.

In addition, the present invention provides a pharmaceutical composition for preventing or treating an aging, comprising vesicles derived from *Lactobacillus rhamnosus* as an active ingredient.

In addition, the present invention provides a food composition for preventing or alleviating an aging, comprising vesicles derived from *Lactobacillus rhamnosus* as an active ingredient.

In addition, the present invention provides a cosmetic composition for preventing or alleviating an aging, comprising vesicles derived from *Lactobacillus rhamnosus* as an active ingredient.

In addition, the present invention provides a method for preventing or treating one or more selected from the group consisting of immune diseases and cellular aging-related diseases, the method comprising administering a composition comprising vesicles derived from *Lactobacillus rhamnosus* as an active ingredient to a subject in need thereof.

In addition, the present invention provides a use of a composition comprising vesicles derived from *Lactobacillus rhamnosus* as an active ingredient for preventing or treating one or more selected from the group consisting of immune diseases and cellular aging-related diseases.

In addition, the present invention provides a use of vesicles derived from *Lactobacillus rhamnosus* for manufacturing a therapeutic agent for treating one or more selected from the group consisting of immune diseases and cellular aging-related diseases.

In addition, the present invention provides a method for preventing or treating an aging, the method comprising administering a composition comprising vesicles derived from *Lactobacillus rhamnosus* as an active ingredient to a subject in need thereof.

In addition, the present invention provides a use of a composition comprising vesicles derived from *Lactobacillus rhamnosus* as an active ingredient for preventing or treating an aging.

In addition, the present invention provides a use of vesicles derived from *Lactobacillus rhamnosus* for manufacturing a therapeutic agent for treating an aging.

### [Advantageous Effects]

It was confirmed that when vesicles derived from *Lactobacillus rhamnosus* bacteria according to the present invention were orally administered to a mouse model of a disease related to cellular aging caused by a high-fat diet, the body weight gain was suppressed, and renal and pulmonary functions were improved. In addition, it was confirmed that the vesicle activates AMPK, which is a key signal that induces cellular homeostasis through autophagy in an energy metabolism stress situation where ATP production does not occur well.

Furthermore, when the vesicles were orally administered to a mouse model of a diseases related to immune dysfunction induced by poly(I:C), which is a viral pathogenic factor, the systemic inflammatory responses caused by immune hypersensitivity reactions were inhibited, and locally, pulmonary inflammation due to immune hypersensitivity reactions caused by viral causative factors was significantly inhibited. In addition, it was confirmed that the immune hypersensitivity reactions due to the viral causative factors described above are controlled by the vesicle acting to inhibit the expression of an NF-κB signal and inducible NOS (iNOS), which is a downstream signal thereof.

Therefore, the *Lactobacillus rhamnosus-derived* vesicle according to the present invention is expected to be effectively used in the development of a drug or health functional food for preventing, alleviating, or treating immune diseases including viral infectious diseases, allergic immune diseases, and autoimmune diseases, and cellular aging-related diseases.

### [Description of Drawings]

FIG. 1 shows the experimental protocol for evaluating a therapeutic effect on cellular aging-related diseases by orally administering *Lactobacillus rhamnosus-*derived vesicles to a mouse model of a cellular aging-related disease induced by a high-fat diet.
FIG. 2 shows the results of evaluating a weight-decreasing effect by orally administering *Lactobacillus rhamnosus-derived* vesicles to a mouse model of a cellular aging-related disease induced by a high-fat diet.
FIGS. 3A and 3B show the results of confirming renal function test (BUN, creatinine) levels in serum by orally administering *Lactobacillus rhamnosus-derived* vesicles to a mouse model of a cellular aging-related disease induced by a high-fat diet.
FIG. 4 shows the results of observing changes in pulmonary function by orally administering *Lactobacillus rhamnosus-derived* vesicles to a mouse model of a cellular aging-related disease induced by a high-fat diet.
FIGS. 5A and 5B show the results of confirming Th17 and Th2-related cytokine levels in airway lavage fluid by orally administering *Lactobacillus rhamnosus-derived* vesicles to a mouse model of a cellular aging-related disease induced by a high-fat diet.
FIG. 6 shows the results of evaluating the effect of *Lactobacillus rhamnosus-*derived vesicles on maintaining cellular homeostasis through AMPK activation in muscle cells.
FIG. 7 shows the experimental protocol for evaluating a therapeutic effect on an immune disease by orally administering *Lactobacillus rhamnosus-derived* vesicles to mice of an immune dysfunction-related disease induced by intranasally administering poly(I:C), which is a causative factor that mimics immune diseases caused by viral infection.
FIGS. 8A and 8B show the results of observing changes in (A) the number of inflammatory cells and (B) the number of macrophages and lymphocytes in airway lavage fluid by orally administering *Lactobacillus rhamnosus-derived* vesicles to a mouse model of an immune disease induced by intranasally administering poly(I:C).
FIGS. 9A and 9B show images of the pulmonary histological changes observed through H&E staining (FIG. 9A) and the number of immune cells infiltrated around the respiratory tract (FIG. 9B) by orally administering *Lactobacillus rhamnosus-*derived vesicles to a mouse model of an immune disease induced by intranasally administering poly(I:C).
FIG. 10 shows the results of confirming an hs-CRP level, which is a blood inflammatory indicator, by orally administering *Lactobacillus rhamnosus-derived* vesicles to a mouse model of an immune disease induced by intranasally administering poly(I:C).
FIGS. 11A and 11B show the results of confirming inflammatory cytokine (IFN-γ, IL-6) levels in airway lavage fluid by orally administering *Lactobacillus rhamnosus-derived* vesicles to a mouse model of an immune disease induced by intranasally administering poly(I:C).
FIGS. 12A and 12B show the results of confirming inflammatory cytokine (IFN-γ, IL-6) levels secreted from T cells after stimulating T cells with anti-CD3 and anti-CD28 antibodies in splenic T cells obtained by orally administering *Lactobacillus rhamnosus-derived* vesicles to a mouse model of an immune disease induced by intranasally administering poly(I:C).
FIG. 13 shows the results of confirming the expression of NF-κB and iNOS in a pulmonary tissue obtained after orally administering *Lactobacillus rhamnosus-*derived vesicles to a mouse model of an immune disease induced by intranasally administering poly(I:C).
FIG. 14 is a diagram summarizing the action mechanism of a *Lactobacillus rhamnosus* on immune diseases and metabolic diseases at the level of molecular biology.

### [Best Mode]

The present invention relates to *Lactobacillus rhamnosus-derived* vesicles and uses thereof.

Hereinafter, the present invention will be described in detail.

The present inventors have confirmed that when *Lactobacillus rhamnosus* bacteria were cultured and vesicles were isolated from the culture medium and orally administered to chronic disease mice in which cellular aging had been induced by a high-fat diet, the body weight gain was suppressed, and renal and pulmonary functions were improved, and also confirmed that this action is related to the inhibition of hypersensitivity reactions caused by Th17 and Th2 cells (see Examples 2 and 3).

In addition, it was confirmed that the vesicle of the present invention activates AMPK, which is a key signal that induces cellular homeostasis through autophagy in energy metabolism stress situations where ATP production does not occur well (see Example 4).

In addition, it was confirmed that when the vesicles were orally administered to a mouse model of an immune disease induced by poly(I:C), which is a viral pathogenic factor, the systemic inflammatory response was inhibited, and locally, pulmonary inflammatory caused by the viral causative factors was significantly inhibited (see Examples 5 and 6).

In addition, it was confirmed that the immune hypersensitivity reactions due to the viral causative factors described above are controlled by the vesicle acting to inhibit the expression of an NF-xB signal and inducible NOS (iNOS), which is a downstream signal thereof (see Example 7). Therefore, it was confirmed that the *Lactobacillus rhamnosus-derived* vesicle according to the present invention may be effectively used as a composition for preventing or treating immune diseases and cellular aging-related neurological diseases.

Thus, the present invention provides a pharmaceutical composition for preventing or treating one or more selected from the group consisting of immune diseases and cellular aging-related diseases, comprising vesicles derived from *Lactobacillus rhamnosus* as an active ingredient.

As used herein, the term "extracellular vesicle" or "vesicle" refers to a structure formed of a nano-sized membrane secreted from various bacteria, and in the present invention, the vesicle collectively refers to all structures made of membranes that are naturally secreted from *Lactobacillus rhamnosus* or artificially produced.

The vesicles may be isolated by heat treatment or autoclaving during *Lactobacillus rhamnosus* culture, or using one or more methods selected from the group consisting of centrifugation, ultracentrifugation, autoclaving, extrusion, sonication, cell lysis, homogenization, freezing-thawing, electroporation, mechanical degradation, chemical treatment, filtration with a filter, gel filtration chromatography, pre-flow electrophoresis, and capillary electrophoresis of the cell culture. In addition, for isolation, washing for removing impurities, and concentration of the obtained vesicles may be further performed.

The method of isolating vesicles from a culture medium or fermented food of *Lactobacillus rhamnosus* of the present invention is not particularly limited as long as it provides vesicles. For example, methods such as centrifugation, ultra-high-speed centrifugation, filtration through filter, gel filtration chromatography, free-flow electrophoresis, or capillary electrophoresis, and combinations thereof may be used to isolate vesicles, and processes such as washing for removing impurities and concentration of the obtained vesicles may be further included.

The vesicles of the present invention may be isolated from a culture medium of *Lactobacillus rhamnosus* or from food manufactured by adding *Lactobacillus rhamnosus,* and may be naturally secreted from *Lactobacillus rhamnosus* or artificially produced, but are not limited thereto.

In the present invention, the vesicles isolated by the methods described above may have an average diameter of 10-1000 nm, 10-900 nm, 10-800 nm, 10-700 nm, 10-600 nm, 10-500 nm, 10-400 nm, 10 -300 nm, 10-220 nm, 10-200 nm, 10-100 nm, 10-90 nm, 10-80 nm, 10-70 nm, 10-60 nm, 10-50 nm, 10-40 nm, or 20 to 40 nm, but it is not limited thereto.

The term "immune disease" used in the present invention collectively refers to diseases caused by immune dysfunction in the living body. Generally, in an immune disease, inflammation is caused by pathogenic causative factors, and as a result, apoptosis and inflammatory cell infiltration by inflammatory mediators are induced. In addition, "immune dysfunction-related disease" or "immune disease" of the present invention collectively refers to diseases caused by immune dysfunction characterized by immune hypersensitivity reactions caused by external factors or internal factors. Generally, viral infectious diseases and allergic immune diseases occur due to immune dysfunction characterized by hypersensitivity reactions to external factors such as viruses and allergens, and autoimmune diseases occur due to immune dysfunction to internal causative factors such as autoantigens. The immune dysfunction-related diseases or immune diseases include viral infectious diseases, allergic immune diseases, and autoimmune diseases.

In the present invention, the viral infectious disease may be one or more selected from the group consisting of viral pneumonia, pulmonary tuberculosis, tuberculosis, the common cold, influenza, respiratory tract infections, rhinitis, nasopharyngitis, otitis media, bronchitis, lymphadenitis, parotitis, lymphadenitis, cheilitis, stomatitis, arthritis, myositis, dermatitis, vasculitis, gingivitis, periodontitis, keratitis, conjunctivitis, wound infections, peritonitis, hepatitis, osteomyelitis, cellulitis, meningitis, encephalitis, pyencephalus, encephalomyelitis, meningitis, osteomyelitis, nephritis, carditis, endocarditis, enteritis, gastritis, esophagitis, duodenitis, colitis, urethritis, cystitis, vaginitis, cervicitis, salpingitis, erythema infectiosum, bacillary dysentery, abscesses and ulcers, bacteremia, diarrhea, dysentery, gastroenteritis, urogenital abscesses, infections of open wounds or wounds, purulent inflammation, abscesses, boils, pyoderma, impetigo, folliculitis, cellulitis, postoperative wound infections, skin laceration syndrome, skin burn syndrome, thrombotic thrombocytopenia, hemolytic uremic syndrome, renal failure, pyelonephritis, glomerulonephritis, nervous system abscesses, tympanitis, sinusitis, pharyngitis, tonsillitis, mastoiditis, cellulitis, odontogenic infections, dacryocystitis, pleurisy, abdominal abscesses, hepatic abscesses, cholecystitis, splenic abscesses, pericarditis, myocarditis, placentitis, amnionitis, mastitis, septic shock, systemic inflammatory response syndrome (SIRS), multiple organ dysfunction syndrome (MODS), thromboembolism, shingles, and Meniere's disease, but is not limited thereto.

In the present invention, the autoimmune disease may be one or more selected from the group consisting of sepsis, arteriosclerosis, bacteremia, systemic inflammatory response syndrome, multiple organ dysfunction, osteoporosis, periodontitis, systemic lupus erythematosus, rheumatoid arthritis, osteoarthritis, juvenile chronic arthritis, spondyloarthritis, multiple sclerosis, systemic sclerosis, idiopathic inflammatory muscle disorders, Sjogren's syndrome, systemic vasculitis, sarcoidosis, autoimmune hemolytic anemia, autoimmune thrombocytopenia, thyroiditis, diabetes mellitus, immune-mediated renal disease, demyelinating diseases of the central nervous system or the peripheral nervous system, idiopathic demyelinating polyneuritis, Guillain-Barre syndrome, chronic inflammatory demyelinating polyneuritis, hepatobiliary diseases, infectious or autoimmune chronic active hepatitis, primary biliary cirrhosis, granulomatous hepatitis, sclerosing cholangitis, inflammatory bowel disease (IBD), ulcerative colitis, Crohn's disease, irritable bowel syndrome, gluten-sensitive bowel disease, Whipple's disease, autoimmune or immune-mediated skin diseases, blistering skin diseases, erythema multiforme, contact dermatitis, psoriasis, allergic immune diseases, asthma, allergic rhinitis, atopic dermatitis, food intolerances, acne, hives, pulmonary immune diseases, eosinophilic pneumonia, idiopathic pulmonary fibrosis, and hypersensitivity pneumonitis, but is not limited thereto.

In the present invention, the allergic immune disease may be one or more selected from the group consisting of atopic dermatitis, allergic conjunctivitis, allergic rhinitis, allergic asthma, hypersensitivity pneumonitis, food allergies, celiac disease, irritable bowel syndrome, and anaphylaxis, but is not limited thereto.

The term "cellular aging-related disease" as used in the present invention collectively refers to diseases that occur when cellular aging is accelerated by energy metabolism stress in the living body due to overnutrition. The cellular aging-related disease include: one or more cardiovascular diseases selected from the group consisting of hypertension, arteriosclerosis, angina pectoris, hyperlipidemia, cardiac failure, myocardial infarction, cardiomyopathy, thromboembolism, and stroke;
one or more hepato-biliary-pancreatic diseases selected from the group consisting of non-alcoholic steatohepatitis (NASH), alcoholic steatohepatitis, liver cirrhosis, cholangitis, cholecystitis, and pancreatitis;
one or more renal diseases selected from the group consisting of glomerulonephritis, and chronic nephropathy;
one or more musculoskeletal diseases selected from the group consisting of atony, muscular atrophy, muscular dystrophy, myasthenia, cachexia, gout, sarcopenia, osteoporosis, Paget's disease, rheumatoid arthritis, and osteoarthritis; and
one or more neurological diseases selected from the group consisting of mild cognitive impairment, dementia, Alzheimer's disease, Parkinson's disease, Huntington's disease, amyotrophic lateral sclerosis (ALS), Batten disease, Kearns-Sayre syndrome (KSS), chronic progressive external ophthalmoplegia (CPEO), mitochondrial encephalomyopathy with lactic acidosis and stroke-like episodes (MELAS) syndrome, myoclonic epilepsy with ragged-red fibers (MERRF) syndrome, neurogenic weakness with ataxia and retinitis pigmentosa (NARP) syndrome, Leigh syndrome (LS), mitochondrial recessive ataxia syndrome (MIRAS), dementia with Lewy bodies (DLB), multi-infarct dementia (MID), frontotemporal lobar degeneration (FTLD), Pick's disease, corticobasal degeneration (CBD), progressive supranuclear palsy (PSP), aging-related macular degeneration (AMD), dysosmia, deafness, and diabetic retinopathy.

The amount of the vesicles in the composition of the present invention may be appropriately adjusted depending on the symptoms of a disease, the degree of progression of symptoms, the condition of a patient, and the like, and may range from, for example, 0.0001 wt% to 99.9 wt% or 0.001 wt% to 50 wt% with respect to a total weight of the composition, but the present invention is not limited thereto. The amount ratio is a value based on the amount of dried product from which a solvent is removed.

The pharmaceutical composition according to the present invention may further include a suitable carrier, excipient, and diluent which are commonly used in the preparation of pharmaceutical compositions. The excipient may be, for example, one or more selected from the group consisting of a diluent, a binder, a disintegrant, a lubricant, an adsorbent, a humectant, a film-coating material, and a controlled release additive.

The pharmaceutical composition according to the present invention may be used by being formulated, according to commonly used methods, into a form such as powders, granules, sustained-release-type granules, enteric granules, liquids, eye drops, elixirs, emulsions, suspensions, spirits, troches, aromatic water, lemonades, tablets, sustained-release-type tablets, enteric tablets, sublingual tablets, hard capsules, soft capsules, sustained-release-type capsules, enteric capsules, pills, tinctures, soft extracts, dry extracts, fluid extracts, injections, capsules, perfusates, or a preparation for external use, such as plasters, lotions, pastes, sprays, inhalants, patches, sterile injectable solutions, or aerosols. The preparation for external use may have a formulation such as creams, gels, patches, sprays, ointments, plasters, lotions, liniments, pastes, or cataplasmas.

As the carrier, the excipient, and the diluent that may be included in the pharmaceutical composition according to the present invention, lactose, dextrose, sucrose, oligosaccharides, sorbitol, mannitol, xylitol, erythritol, maltitol, starch, acacia rubber, alginate, gelatin, calcium phosphate, calcium silicate, cellulose, methyl cellulose, microcrystalline cellulose, polyvinylpyrrolidone, water, methyl hydroxybenzoate, propyl hydroxybenzoate, talc, magnesium stearate, and mineral oil may be used.

For formulation, commonly used diluents or excipients such as fillers, thickeners, binders, wetting agents, disintegrants, and surfactants are used.

As additives of tablets, powders, granules, capsules, pills, and troches according to the present invention, excipients such as corn starch, potato starch, wheat starch, lactose, white sugar, glucose, fructose, D-mannitol, precipitated calcium carbonate, synthetic aluminum silicate, dibasic calcium phosphate, calcium sulfate, sodium chloride, sodium hydrogen carbonate, purified lanolin, microcrystalline cellulose, dextrin, sodium alginate, methyl cellulose, sodium carboxymethylcellulose, kaolin, urea, colloidal silica gel, hydroxypropyl starch, hydroxypropyl methylcellulose (HPMC), HPMC 1928, HPMC 2208, HPMC 2906, HPMC 2910, propylene glycol, casein, calcium lactate, and *Primojel*^{®}; and binders such as gelatin, Arabic gum, ethanol, agar powder, cellulose acetate phthalate, carboxymethylcellulose, calcium carboxymethylcellulose, glucose, purified water, sodium caseinate, glycerin, stearic acid, sodium carboxymethylcellulose, sodium methylcellulose, methylcellulose, microcrystalline cellulose, dextrin, hydroxycellulose, hydroxypropyl starch, hydroxymethylcellulose, purified shellac, starch, hydroxypropyl cellulose, hydroxypropyl methylcellulose, polyvinyl alcohol, and polyvinylpyrrolidone may be used, and disintegrants such as hydroxypropyl methylcellulose, corn starch, agar powder, methylcellulose, bentonite, hydroxypropyl starch, sodium carboxymethylcellulose, sodium alginate, calcium carboxymethylcellulose, calcium citrate, sodium lauryl sulfate, silicic anhydride, 1-hydroxypropylcellulose, dextran, ion-exchange resin, polyvinyl acetate, formaldehyde-treated casein and gelatin, alginic acid, amylose, guar gum, sodium bicarbonate, polyvinylpyrrolidone, calcium phosphate, gelled starch, Arabic gum, amylopectin, pectin, sodium polyphosphate, ethyl cellulose, white sugar, magnesium aluminum silicate, a di-sorbitol solution, and light anhydrous silicic acid; and lubricants such as calcium stearate, magnesium stearate, stearic acid, hydrogenated vegetable oil, talc, lycopodium powder, kaolin, Vaseline, sodium stearate, cacao butter, sodium salicylate, magnesium salicylate, polyethylene glycol (PEG) 4000, PEG 6000, liquid paraffin, hydrogenated soybean oil (Lubri wax), aluminum stearate, zinc stearate, sodium lauryl sulfate, magnesium oxide, Macrogol, synthetic aluminum silicate, silicic anhydride, higher fatty acids, higher alcohols, silicone oil, paraffin oil, polyethylene glycol fatty acid ether, starch, sodium chloride, sodium acetate, sodium oleate, dl-leucine, and light anhydrous silicic acid may be used.

As additives of liquids according to the present invention, water, dilute hydrochloric acid, dilute sulfuric acid, sodium citrate, monostearic acid sucrose, polyoxyethylene sorbitol fatty acid esters (twin esters), polyoxyethylene monoalkyl ethers, lanolin ethers, lanolin esters, acetic acid, hydrochloric acid, ammonia water, ammonium carbonate, potassium hydroxide, sodium hydroxide, prolamine, polyvinylpyrrolidone, ethylcellulose, and sodium carboxymethylcellulose may be used.

In syrups according to the present invention, a white sugar solution, other sugars or sweeteners, and the like may be used, and as necessary, a fragrance, a colorant, a preservative, a stabilizer, a suspending agent, an emulsifier, a viscous agent, or the like may be used.

In emulsions according to the present invention, purified water may be used, and as necessary, an emulsifier, a preservative, a stabilizer, a fragrance, or the like may be used.

In suspensions according to the present invention, suspending agents such as acacia, tragacanth, methylcellulose, carboxymethylcellulose, sodium carboxymethylcellulose, microcrystalline cellulose, sodium alginate, hydroxypropyl methylcellulose (HPMC), HPMC 1828, HPMC 2906, HPMC 2910, and the like may be used, and as necessary, a surfactant, a preservative, a stabilizer, a colorant, and a fragrance may be used.

Injections according to the present invention may include: solvents such as distilled water for injection, a 0.9% sodium chloride solution, Ringer's solution, a dextrose solution, a dextrose+sodium chloride solution, PEG, lactated Ringer's solution, ethanol, propylene glycol, non-volatile oil-sesame oil, cottonseed oil, peanut oil, soybean oil, corn oil, ethyl oleate, isopropyl myristate, and benzene benzoate; cosolvents such as sodium benzoate, sodium salicylate, sodium acetate, urea, urethane, monoethylacetamide, butazolidine, propylene glycol, the Tween series, amide nicotinate, hexamine, and dimethylacetamide; buffers such as weak acids and salts thereof (acetic acid and sodium acetate), weak bases and salts thereof (ammonia and ammonium acetate), organic compounds, proteins, albumin, peptone, and gums; isotonic agents such as sodium chloride; stabilizers such as sodium bisulfite (NaHSO₃) carbon dioxide gas, sodium metabisulfite (Na₂S₂O₅), sodium sulfite (Na₂SO₃), nitrogen gas (N₂), and ethylenediamine tetraacetic acid; sulfating agents such as 0.1% sodium bisulfide, sodium formaldehyde sulfoxylate, thiourea, disodium ethylenediaminetetraacetate, and acetone sodium bisulfite; a pain relief agent such as benzyl alcohol, chlorobutanol, procaine hydrochloride, glucose, and calcium gluconate; and suspending agents such as sodium CMC, sodium alginate, Tween 80, and aluminum monostearate.

In suppositories according to the present invention, bases such as cacao butter, lanolin, Witepsol, polyethylene glycol, glycerogelatin, methylcellulose, carboxymethylcellulose, a mixture of stearic acid and oleic acid, Subanal, cottonseed oil, peanut oil, palm oil, cacao butter + cholesterol, lecithin, lanette wax, glycerol monostearate, Tween or span, imhausen, monolan(propylene glycol monostearate), glycerin, Adeps solidus, buytyrum Tego-G, cebes Pharma 16, hexalide base 95, cotomar, Hydrokote SP, S-70-XXA, S-70-XX75(S-70-XX95), Hydrokote 25, Hydrokote 711, idropostal, massa estrarium (A, AS, B, C, D, E, I, T), masa-MF, masupol, masupol-15, neosuppostal-N, paramount-B, supposiro OSI, OSIX, A, B, C, D, H, L, suppository base IV types AB, B, A, BC, BBG, E, BGF, C, D, 299, suppostal N, Es, Wecoby W, R, S, M, Fs, and tegester triglyceride matter (TG-95, MA, 57) may be used.

Solid preparations for oral administration include tablets, pills, powders, granules, capsules, and the like, and such solid preparations are formulated by mixing the composition with at least one excipient, e.g., starch, calcium carbonate, sucrose, lactose, gelatin, and the like. In addition to simple excipients, lubricants such as magnesium stearate and talc are also used.

Examples of liquid preparations for oral administration include suspensions, liquids for internal use, emulsions, syrups, and the like, and these liquid preparations may include, in addition to simple commonly used diluents, such as water and liquid paraffin, various types of excipients, for example, a wetting agent, a sweetener, a fragrance, a preservative, and the like. Preparations for parenteral administration include an aqueous sterile solution, a non-aqueous solvent, a suspension, an emulsion, a freeze-dried preparation, and a suppository. Non-limiting examples of the non-aqueous solvent and the suspension include propylene glycol, polyethylene glycol, a vegetable oil such as olive oil, and an injectable ester such as ethyl oleate.

The pharmaceutical composition according to the present invention is administered in a pharmaceutically effective amount. In the present invention, "the pharmaceutically effective amount" refers to an amount sufficient to treat diseases at a reasonable benefit/risk ratio applicable to medical treatment, and an effective dosage level may be determined according to factors including types of diseases of patients, the severity of disease, the activity of drugs, sensitivity to drugs, administration time, administration route, excretion rate, treatment period, and simultaneously used drugs, and factors well known in other medical fields.

The composition according to the present invention may be administered as an individual therapeutic agent or in combination with other therapeutic agents, may be administered sequentially or simultaneously with therapeutic agents in the related art, and may be administered in a single dose or multiple doses. It is important to administer the composition in a minimum amount that can obtain the maximum effect without any side effects, in consideration of all the aforementioned factors, and this may be easily determined by those of ordinary skill in the art.

The pharmaceutical composition of the present invention may be administered to a subject via various routes. All administration methods can be predicted, and the pharmaceutical composition may be administered via, for example, oral administration, subcutaneous injection, intraperitoneal injection, intravenous injection, intramuscular injection, intrathecal (space around the spinal cord) injection, sublingual administration, administration via the buccal mucosa, intrarectal insertion, intravaginal insertion, ocular administration, intra-aural administration, intranasal administration, inhalation, spraying via the mouth or nose, transdermal administration, percutaneous administration, or the like.

The pharmaceutical composition of the present invention is determined depending on the type of a drug, which is an active ingredient, along with various related factors such as a disease to be treated, administration route, the age, gender, and body weight of a patient, and the severity of diseases. Specifically, the effective amount of the composition according to the present invention may vary depending on the patient's age, sex, and body weight, and generally, 0.001 to 150 mg of the composition and preferably, 0.01 to 100 mg of the composition, per 1 kg of the body weight, may be administered daily or every other day or may be administered once to three times a day. However, since the effective amount may be increased or decreased depending on the administration route, the severity of obesity, gender, body weight, age, and the like, the dosage is not intended to limit the scope of the present invention in any way.

In addition, the present invention provides a method for preventing or treating one or more selected from the group consisting of immune diseases and cellular aging-related diseases, the method comprising administering a composition comprising vesicles derived from *Lactobacillus rhamnosus* as an active ingredient to a subject in need thereof.

In addition, the present invention provides a use of a composition comprising vesicles derived from *Lactobacillus rhamnosus* as an active ingredient for preventing or treating one or more selected from the group consisting of immune diseases and cellular aging-related diseases.

In addition, the present invention provides a use of vesicles derived from *Lactobacillus rhamnosus* for manufacturing a therapeutic agent for treating one or more selected from the group consisting of immune diseases and cellular aging-related diseases

As used herein, the "subject" refers to a subject in need of treatment of a disease, and more specifically, refers to a mammal such as a human or a non-human primate, a mouse, a rat, a dog, a cat, a horse, and a cow, but the present invention is not limited thereto.

As used herein, the "administration" refers to providing a subject with a predetermined composition of the present invention by using an arbitrary appropriate method.

The term "prevention" as used herein means all actions that inhibit or delay the onset of a target disease. The term "treatment" as used herein means all actions that alleviate or beneficially change a target disease and abnormal metabolic symptoms caused thereby via administration of the pharmaceutical composition according to the present invention. The term "alleviation" as used herein means all actions that reduce the degree of parameters related to a target disease, e.g., symptoms via administration of the composition according to the present invention.

In addition, the present invention provides a food composition for preventing or alleviating one or more selected from the group consisting of immune diseases and cellular aging-related diseases, comprising vesicles derived from *Lactobacillus rhamnosus* as an active ingredient.

The food composition may be a health functional food composition, but is not limited thereto.

The vesicles according to the present invention may be used by adding the vesicles as is to food or may be used together with other foods or food ingredients, but may be appropriately used according to a typical method. The mixed amount of the active ingredient may be suitably determined depending on the purpose of use thereof (for prevention or alleviation). In general, when a food or beverage is prepared, the composition of the present invention is added in an amount of 15 wt% or less, preferably 10 wt% or less based on the raw materials. However, for long-term intake for the purpose of health and hygiene or for the purpose of health control, the amount may be less than the above-mentioned range, and the vesicles have no problem in terms of stability, so the active ingredient may be used in an amount more than the above-mentioned range.

The type of food is not particularly limited. Examples of food to which the material may be added include meats, sausage, bread, chocolate, candies, snacks, confectioneries, pizza, instant noodles, other noodles, gums, dairy products including ice creams, various soups, beverages, tea, drinks, alcoholic beverages, vitamin complexes, and the like, and include all health functional foods in a typical sense.

The health beverage composition according to the present invention may contain various flavors or natural carbohydrates, and the like as additional ingredients as in a typical beverage. The above-described natural carbohydrates may be monosaccharides such as glucose and fructose, disaccharides such as maltose and sucrose, polysaccharides such as dextrin and cyclodextrin, and sugar alcohols such as xylitol, sorbitol, and erythritol. As a sweetener, it is possible to use a natural sweetener such as thaumatin and stevia extract, a synthetic sweetener such as saccharin and aspartame, and the like. The proportion of the natural carbohydrates is generally about 0.01 to 0.20 g, or about 0.04 to 0.10 g per 100 ml of the composition of the present invention.

In addition to the aforementioned ingredients, the composition of the present invention may contain various nutrients, vitamins, electrolytes, flavors, colorants, pectic acids and salts thereof, alginic acid and salts thereof, organic acids, protective colloid thickeners, pH adjusters, stabilizers, preservatives, glycerin, alcohols, carbonating agents used in carbonated drinks, and the like. In addition, the composition of the present invention may contain flesh for preparing natural fruit juice, fruit juice drinks, and vegetable drinks. These ingredients may be used either alone or in combinations thereof. The proportion of these additives is not significantly important, but is generally selected within a range of 0.01 to 0.20 part by weight per 100 parts by weight of the composition of the present invention.

In addition, the present invention provides a pharmaceutical composition for preventing or treating an aging or an aging-related disease, comprising vesicles derived from *Lactobacillus rhamnosus* as an active ingredient.

In addition, the present invention provides a food composition for preventing or alleviating an aging or an aging-related disease, comprising vesicles derived from *Lactobacillus rhamnosus* as an active ingredient.

In addition, the present invention provides a cosmetic composition for preventing or alleviating an aging, comprising vesicles derived from *Lactobacillus rhamnosus* as an active ingredient.

In addition, the present invention provides a method for preventing or treating an aging, the method comprising administering a composition comprising vesicles derived from *Lactobacillus rhamnosus* as an active ingredient to a subject in need thereof.

In addition, the present invention provides a use of a composition comprising vesicles derived from *Lactobacillus rhamnosus* as an active ingredient for preventing or treating an aging.

In addition, the present invention provides a use of vesicles derived from *Lactobacillus rhamnosus* for manufacturing a therapeutic agent for treating an aging.

In the present invention, "aging" collectively refers to all physiological changes in the body that occur over time and means life phenomena that occur in extremely various ways depending on individuals due to numerous factors. When aging phenomena are considered specifically, changes in the function of each component organ and tissue occur, and the aging of an individual is ultimately caused by the aging of the cells that constitute the individual. In the present invention, the aging may be due to cellular aging of the brain, liver, lungs, kidneys, muscles, skin, or these organs, but is not limited thereto.

In the present invention, "aging-related disease" may be a disease caused by aging of cells that are present in organs such as the brain, liver, lungs, kidneys, heart, muscles, and skin, but is not limited thereto.

A formulation for the cosmetic composition according to the present invention may include a skin lotion, a skin softener, a skin toner, an astringent, a lotion, a milk lotion, a moisturizing lotion, a nourishing lotion, a massage cream, a nourishing cream, a mist, a moisturizing cream, a hand cream, a hand lotion, a foundation, an essence, a nourishing essence, a pack, soap, a cleansing foam, a cleansing lotion, a cleansing cream, a cleansing oil, a cleansing balm, a body lotion or a body cleanser.

A cosmetic composition of the present invention may further include a composition selected from the group consisting of water-soluble vitamins, oil-soluble vitamins, polymer peptides, polymeric polysaccharides, and sphingolipids.

The water-soluble vitamin may be any substance that is blendable with cosmetics, but examples thereof include vitamin B1, vitamin B2, vitamin B6, pyridoxine, pyridoxine hydrochloride, vitamin B 12, pantothenic acid, nicotinic acid, nicotinic acid amide, folic acid, vitamin C, vitamin H, and the like, and salts thereof (thiamine hydrochloride, sodium ascorbate, and the like) or derivatives thereof (sodium ascorbic acid-2-phosphate, magnesium ascorbic acid-2-phosphate, and the like) are also included in water-soluble vitamins that may be used in the present invention. These water-soluble vitamins may be obtained by a conventional method such as microbial transformation, purification from a microbial culture, an enzyme method, or a chemical synthesis method.

The oil-soluble vitamins may be any substance that is blendable with cosmetics, but examples thereof include vitamin A, carotene, vitamin D2, vitamin D3, vitamin E (d1-α-tocopherol, d-α-tocopherol), or the like, and derivatives thereof (e.g., ascorbyl palmitate, ascorbyl stearate, ascorbyl dipalmitate, d1-α-tocopherol acetate, d1-α-tocopherol nicotinate, vitamin E, DL-pantothenyl alcohol, D-pantothenyl alcohol, pantothenyl ethylether) may also be included in the oil-soluble vitamins used in the present invention. These oil-soluble vitamins may be obtained by a conventional method such as microbial transformation, purification from a microbial culture, or enzymatic or chemical synthesis.

The polymer peptides may be any substance that is blendable with cosmetics, but examples thereof may include collagen, hydrolyzed collagen, gelatin, elastin, hydrolyzed elastin, and keratin. The polymer peptides may be purified and obtained by any conventional method such as purification from a microbial culture, an enzyme method, or a chemical synthesis method, or may generally be used by being purified from natural substances such as the dermis of a pig, a cow, or the like and silk fiber of silkworms.

The polymeric polysaccharides may be any substance that is blendable with cosmetics, and examples thereof may include hydroxyethyl cellulose, xanthan gum, sodium hyaluronate, and chondroitin sulfate or salts thereof (sodium salts). For example, chondroitin sulfate or salts thereof may generally be purified from mammals or fish and used.

The sphingolipids may be any substance that is blendable with cosmetics, and examples thereof may include ceramide, phytosphingosine, and sphingoglycolipid. The sphingolipids may be purified, by a conventional method, from mammals, fish, shellfish, yeast, or plants, or may be obtained by a chemical synthesis method.

The cosmetic composition of the present invention may include, as necessary, other ingredients mixed in conventional cosmetics along with the above essential ingredients.

Examples of additional ingredients to be mixed may include lipid components, a humectant, an emollient, a surfactant, organic and inorganic pigments, organic powder, a UV absorbent, a preservative, a sanitizer, an antioxidant, a plant extract, a pH adjuster, alcohol, pigments, flavors, a blood circulation promoter, a cooling agent, an anti-diaphoretic, and purified water.

The lipid components may include, for example, ester lipids, hydrocarbon lipids, silicone lipids, fluorine lipids, animal fats, vegetable oil, or the like.

The ester lipids may include, for example, glyceryl tri 2-ethylhexanoate, cetyl 2-ethylhexanoate, isopropyl myristate, butyl myristate, isopropyl palmitate, ethyl stearate, octyl palmitate, isocetyl isostearate, butyl stearate, ethyl linolate, isopropyl linolate, ethyl oleate, isocetyl myristate, isostearyl myristate, isostearyl palmitate, octyldodecyl myristate, isocetyl isostearate, diethyl sebacate, diisopropyl adipate, isoalkyl neopentanate, tri(capryl, capric acid)glyceryl, trimethylolpropane tri 2-ethylhexanoate, trimethylolpropane triisostearate, pentaerythritol tetra 2-ethylhexanoate, cetyl caprylate, decyl laurate, hexyl laurate, decyl myristate, myristyl myristate, cetyl myristate, stearyl stearate, decyl oleate, cetyl ricinoleate, isostearyl laurate, isotridecyl myristate, isocetyl palmitate, octyl stearate, isocetyl stearate, isodecyl oleate, octyldodecyl oleate, octyldodecyl linolate, isopropyl isostearate, cetostearyl 2-ethylhexanoate, stearyl 2-ethylhexanoate, hexyl isostearate, ethyleneglycol dioctanoate, ethyleneglycol dioleate, propyleneglycol dicaprinate, propyleneglycol di(caprylate, caprinate), propyleneglycol dicaprylate, neopentylglycol dicaprinate, neopentylglycol dioctanoate, glyceryl tricaprylate, glyceryl triundecylate, glyceryl triisopalmitate, glyceryl triisostearate, octyldodecyl neopentanoate, isostearyl octanoate, octyl isononanoate, hexyldecyl neodecanoate, octyldodecyl neodecanoate, isocetyl isostearate, isostearyl isostearate, octyldecyl isostearate, polyglycerin ester oleate, polyglycerin ester isostearate, triisocetyl citrate, triisoalkyl citrate, triisooctyl citrate, lauryl lactate, myristyl lactate, cetyl lactate, octyldecyl lactate, triethyl citrate, acetyltriethyl citrate, acetyltributyl citrate, trioctyl citrate, diisostearyl malate, 2-ethylhexyl hydroxystearate, di 2-ethylhexyl succinate, diisobutyl adipate, diisopropyl sebacate, dioctyl sebacate, cholesteryl stearate, cholesteryl isostearate, cholesteryl hydroxystearate, cholesteryl oleate, dihydrocholesteryl oleate, phytosteryl isostearate, phytosteryl oleate, isocetyl 12-stearoyl hydroxystearate, stearoyl 12-stearoyl hydroxystearate, isostearyl 12-stearoyl hydroxystearate, and the like.

The hydrocarbon lipids may include, for example, squalene, liquid paraffin, alpha-olefin oligomers, isoparaffin, ceresine, paraffin, liquid isoparaffin, polybutene, microcrystalline wax, Vaseline, and the like.

The silicone lipids may include, for example, polymethyl silicon, methylphenyl silicon, methyl cyclopolysiloxane, octamethyl polysiloxane, decamethyl polysiloxane, dodecamethyl cyclosiloxane, dimethylsiloxane/methylcetyloxysiloxane copolymers, dimethylsiloxane/methylstearoxysiloxane copolymers, alkyl-modified silicon oil, amino-modified silicon oil, and the like.

The fluorine lipids may include perfluoropolyether and the like.

The animal or vegetable oil may include avocado oil, almond oil, olive oil, sesame oil, rice bran oil, safflower oil, soybean oil, corn oil, rape flower oil, apricot kernel oil, palm kernel oil, palm oil, castor oil, sunflower oil, grape seed oil, cotton seed oil, coconut oil, tallow nut oil, wheat germ oil, rice germ oil, Shea butter, evening primrose oil, macadamia nut oil, meadow foam seed oil, yolk oil, beef tallow, hemp seed oil, mink oil, orange roughy oil, jojoba oil, candelilla wax, carnauba wax, liquid lanolin, dehydrated castor oil, and the like.

The humectant may include water-soluble low molecular humectants, oil-soluble molecular humectants, water-soluble polymers, oil-soluble polymers, and the like.

The water-soluble low molecular humectants may include serine, glutamine, sorbitol, mannitol, pyrrolidone-sodium carboxylate, glycerin, propylene glycol, 1,3-butylene glycol, ethylene glycol, polyethylene glycol B (degree of polymerization: n=2 or higher), polypropylene glycol (degree of polymerization: n=2 or higher), polyglycerin B (degree of polymerization: n=2 or higher), lactic acid, lactates, and the like.

The oil-soluble low molecular humectants may include cholesterol, cholesterol ester, and the like.

The water-soluble polymers may include carboxyvinyl polymers, polyasparaginic acid salts, tragacanth, xanthan gum, methyl cellulose, hydroxymethyl cellulose, hydroxylethyl cellulose, hydroxypropyl cellulose, carboxymethyl cellulose, water-soluble chitin, chitosan, dextrin, and the like.

The oil-soluble polymers may include, for example, polyvinyl pyrrolidone/eicosen copolymers, polyvinyl pyrrolidone/hexadecene copolymers, nitrocellulose, dextrin fatty acid ester, silicone polymers, and the like.

The emollients may include, for example, long chain cholesterylester acyl glutamate, cholesteryl hydroxystearate, 12-hydroxystearic acid, stearic acid, rosin acid, lanolin fatty acid cholesteryl ester, and the like.

The surfactants may include, for example, non-ionic surfactants, anionic surfactants, cationic surfactants, amphoteric surfactants, and the like.

The non-ionic surfactants may include self-emulsion type glycerin monostearate, propyleneglycol fatty acid ester, glycerin fatty acid ester, polyglycerin fatty acid ester, sorbitan fatty acid ester, polyoxyethylene (POE) sorbitan fatty acid ester, POE sorbit fatty acid ester, POE glycerin fatty acid ester, POE alkylethers, POE fatty acid ester, POE dehydrated castor oil, POE castor oil, polyoxyethylene/polyoxypropylene (POE/POP) copolymers, POE/POP alkylethers, polyether-modified silicone, alkanolamide laurate, alkylamine oxide, hydrated soy phospholipids, and the like.

The anionic surfactants may include fatty acid soap, α-acylsulfonate, alkyl sulfonates, alkylallyl sulfonates, alkylnaphthalene sulfonates, alkyl sulfates, POE alkylether sulfates, alkylamide sulfates, alkyl phosphates, POE alkyl phosphates, alkylamide phosphates, alkyloyl alkyltaurin salts, N-acylamino acid salts, POE alkylether carboxylates, alkyl sulfosuccinates, sodium alkyl sulfoacetates, acylated hydrolyzed collagen peptide salts, perfluoroalkyl ester phosphates, and the like.

The cationic surfactants may include, for example, alkyltrimethylammonium chloride, stearyltrimethylammonium chloride, steraryltrimethylammonium bromide, cetostearyl trimethylammonium chloride, distearyl dimethylammonium chloride, stearylaryl dimethylbenzylammonium chloride, behenyltrimethylammonium bromide, benzalkonium chloride, diethylaminoethylamide stearate, dimethylaminopropylamide stearate, quaternary ammonium salts of lanolin derivatives, and the like.

The amphoteric surfactants may include carboxybetaine, amidebetaine, sulfobetaine, hydroxysulfobetaine, amidesulfobetaine, phosphobetaine, aminocarboxylate, imidazoline derivatives, amideamine-based amphoteric surfactants, and the like.

The organic and inorganic pigments may include: inorganic pigments such as silicic acid, anhydrous silicic acid, magnesium silicate, talc, sericite, mica, kaolin, bengala, clay, bentonite, titanium dioxide-coated mica, bismuth oxychloride, zirconium oxide, magnesium oxide, zinc oxide, titanium oxide, aluminum oxide, calcium sulfate, barium sulfate, magnesium sulfate, calcium carbonate, magnesium carbonate, iron oxide, ultramarine, chromium oxide, chromium hydroxide, calamine, and combinations thereof; organic pigments such as polyamide, polyester, polypropylene, polystyrene, polyurethane, vinyl resin, urea resin, phenol resin, fluorine resin, silicon resin, acryl resin, melamine resin, epoxy resin, polycarbonate resin, divinyl benzene/styrene copolymers, silk powder, cellulose, CI pigment yellow, and CI pigment orange; and composite pigments of inorganic and organic pigments.

The organic powder may include: metallic soap such as calcium stearate; metal salts of alkyl phosphoric acid such as zinc sodium cetylate, zinc laurylate, and calcium laurylate; polymetallic salts of acylamino acid such as calcium N-lauroyl-beta-alanine, zinc N-lauroyl-beta-alanine, and calcium N-lauroylglycine; polymetallic salts of amide sulfonates such as calcium N-lauroyl-taurine and calcium N-palmitoyl-taurine.; N-acyl alkaline amino acids such as N-epsilon-lauroyl-L-lysine, N-epsilon-palmitoyl lysine, N-α-palmitoylol nitin, N-α-lauroyl arginine, and N-α-dehydrated tallow fatty acid acyl arginine; N-acyl polypeptides such as N-lauroyl glycylglycine; α-amino fatty acids such as α-aminocaprylic acid and α-aminolauric acid; polyethylene; polypropylene; nylon; polymethylmethacrylate; polystyrene; divinylbenzene/styrene copolymers; ethylene tetrafluoride; and the like.

The UV absorbents may include para-aminobenzoic acid, ethyl para-aminobenzoate, amyl para-aminobenzoate, octyl para-aminobenzoate, ethyleneglycol salicylate, phenyl salicylate, octyl salcylate, benzyl salicylate, butylphenyl salicylate, homomentyl salicylate, benzyl cinnamate, para-methoxycinnamic acid-2-ethoxylethyl, octyl paramethoxycinnamate, mono-2-ethylhexaneglyceryl diparamethoxycinnamate, isopropyl paramethoxycinnamate, diisopropyl/diisopropyl cinnamic acid ester mixtures, urocanic acid, ethyl urocanate, hydroxymethoxybenzophenone, hydroxymethoxybenzophenone sulfonic acid and salts thereof, dihydroxymethoxybenzophenone, sodium dihydroxymethoxybenzophenone disulfonate, dihydroxybenzophenone, tetrahydroxybenzophenone, 4-tert-butyl-4'-methoxydibenzoylmethane, 2,4,6-trianilino-p-(carbo-2'-ethylhexyl-1'-oxy)-1,3,5-triazine, 2-(2-hydroxy-5-methylphenyl)benzotriazole, and the like.

The sanitizers may include hinokitiol, trichloric acid, trichlorohydroxydiphenylether, chlorohexidine gluconate, phenoxyethanol, resorcine, isopropylmethylphenol, azulene, salicylic acid, zinc pyrithione, benzalkonium chloride, light sensitive element No. 301, sodium mononitroguaiacol, undecylenic acid, and the like.

The antioxidants may include butylhydroxyanisole, propyl gallate, elisorbic acid, and the like.

The pH adjusters may include citric acid, sodium citrate, malic acid, sodium malate, fumaric acid, sodium fumarate, succinic acid, sodium succinate, sodium hydroxide, sodium monohydrophosphate, and the like.

The alcohols may include higher alcohols such as cetyl alcohol.

In addition, additional ingredients to be mixed are not limited to the above examples, and any one of the above ingredients may be mixed within a range that does not adversely affect the objectives and effects of the present invention, but may range from 0.01 wt% to 5 wt% or 0.01 wt% to 3 wt% with respect to the total weight of the composition.

For lotion, paste, cream, or gel preparations of the present invention, as a carrier ingredient, animal fiber, vegetable fiber, wax, paraffin, starch, tragacanth, a cellulose derivative, polyethylene glycol, silicon, bentonite, silica, talc, zinc oxide, or the like may be used.

For powder or spray preparations of the present invention, lactose, talc, silica, aluminum hydroxide, calcium silicate, or polyamide powder may be used as a carrier ingredient. In particular, in the case of spray preparations, the composition may further include a propellant such as chlorofluorohydrocarbon, propane/butane, or dimethyl ether.

For solution or emulsion preparations of the present invention, a solvent, a solubilizing agent, or an emulsifying agent may be used as a carrier ingredient, and the carrier ingredient may be, for example, water, ethanol, isopropanol, ethyl carbonate, ethyl acetate, benzyl alcohol, benzyl benzoate, propylene glycol, 1,3-butyl glycol oil, a glycerol aliphatic ester, polyethylene glycol, or a sorbitan fatty acid ester.

For suspension preparations of the present invention, as a carrier ingredient, a liquid diluent such as water, ethanol, or propylene glycol, a suspending agent such as ethoxylated isostearyl alcohol, polyoxyethylene sorbitol ester, or polyoxyethylene sorbitan ester, micro-crystalline cellulose, aluminum methahydroxide, bentonite, agar, tragacanth, or the like may be used.

For surfactant-containing cleansing preparations of the present invention, as a carrier ingredient, an aliphatic alcohol sulfate, an aliphatic alcohol ether sulfate, a sulfosuccinate monoester, isethionate, imidazolinium derivatives, methyltaurate, sarcosinate, fatty acid amide ether sulfate, alkylamidobetaine, an aliphatic alcohol, a fatty acid glyceride, a fatty acid diethanol amide, vegetable oil, a lanolin derivative, an ethoxylated glycerol fatty acid ester, or the like may be used.

### [Modes of the Invention]

Hereinafter, preferred Examples for helping the understanding of the present invention will be suggested. However, the following Examples are provided only to more easily understand the present invention, and the contents of the present invention are not limited by the following Examples.

### [Examples]

### Example 1. Isolation of Lactobacillus rhamnosus-derived vesicles

To isolate *Lactobacillus rhamnosus-derived* extracellular vesicles (EV), a *Lactobacillus rhamnosus* strain was inoculated into a self-developed medium and cultured in an anaerobic environment. After performing stationary culture at 37°C until the absorbance (OD₆₀₀ₙₘ) became 1.0 to 1.5, re-inoculation into the medium and culturing were performed. Then, the culture medium containing the bacterial cells was recovered and centrifuged at 13,000 g at 4°C for 15 minutes to obtain a supernatant from which the bacterial cells were removed. The obtained supernatant was filtered again by using a 0.22 µm filter, and the filtered supernatant was concentrated to a volume of 50 mL or less using a 100 kDa Pellicon 2 Cassette filter membrane (Merck Millipore) and a MasterFlex pump system (Cole-Parmer). The concentrated supernatant was filtered again through a 0.22 µm filter to isolate *Lactobacillus rhamnosus-derived* vesicles (MDH-003). In the examples described below, experiments were conducted by using the isolated vesicles.

### Example 2. Therapeutic effect of Lactobacillus rhamnosus-derived vesicles in mice with cellular aging-related disease induced by overnutrition

Obesity occurs due to overnutrition, and in obesity, which is a low-level systemic chronic inflammatory condition, abnormal metabolites are produced in the body due to overnutrition, and inflammatory mediators are secreted, thereby causing chronic inflammation. In addition, as AMPK signaling, which maintains cellular homeostasis in an energy metabolism stress situation, is inhibited due to overnutrition, thereby promoting cellular aging, cellular aging-related diseases may occur. In particular, results from many studies have shown that obesity is a risk factor involved in the occurrence of asthma and a decrease in pulmonary function. Therefore, an experiment was performed in the following manner to evaluate a therapeutic effect of the *Lactobacillus rhamnosus-derived* vesicle (MDH-003) in mice in which overnutrition was induced by a high-fat diet.

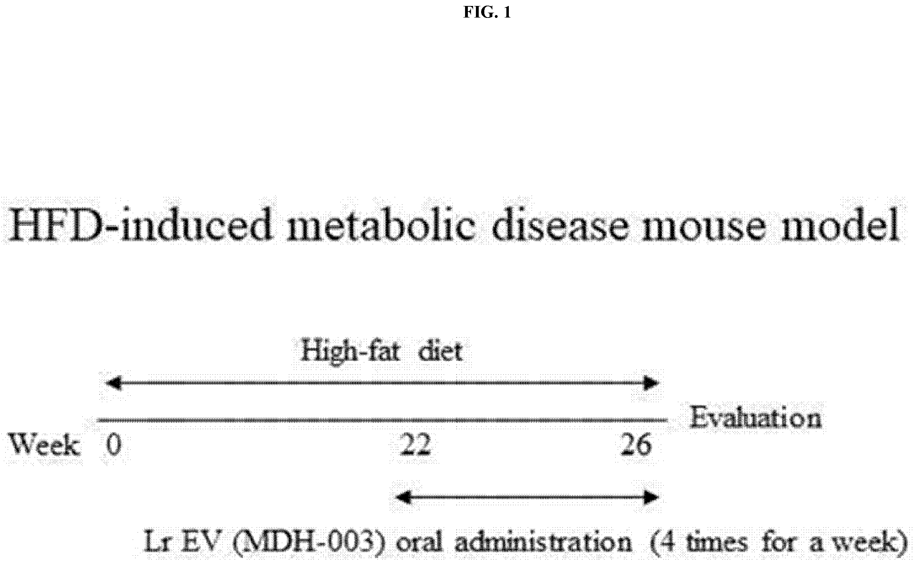

Specifically, after obesity was induced in BALB/c 6-week-old female mice by feeding them a high-fat diet (Harlan, adjusted calories diet) for 22 weeks, a high-fat diet was fed continuously for 4 weeks from week 23 to week 26, and at the same time, *Lactobacillus rhamnosus-derived* vesicles were orally administered four times a week at a dose of 50 µg at a time to perform the evaluation of the efficacy of the *Lactobacillus rhamnosus-derived* vesicles (see FIG. 1).

### 2-1. Confirmation of weight loss effect

The body weight of the high-fat diet mice was measured at week 24 to week 27.

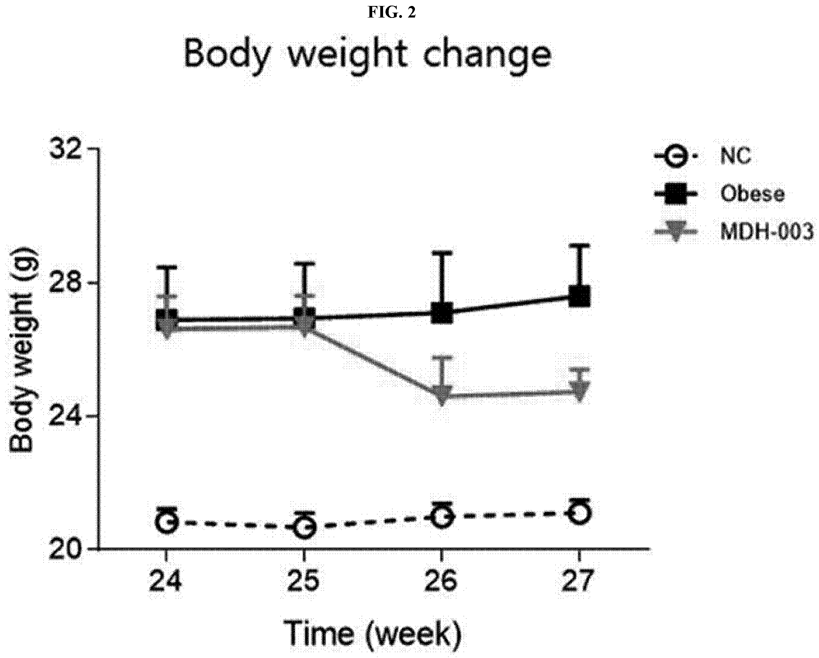

As a result, as shown in FIG. 2, a weight loss effect was observed in the *Lactobacillus rhamnosus-derived* vesicle administration group (MDH-003) compared to the obese group (Obese) from week 26.

### 2-2. Confirmation of renal function improvement effect

Renal function was evaluated through testing blood urea nitrogen (BUN) and creatinine (Cr) levels in serum, which are markers of kidney damage.

Creatinine and BUN in serum were measured using an assay kit (Pointe Scientific, Lincoln Park, MI), which is currently commercially available.

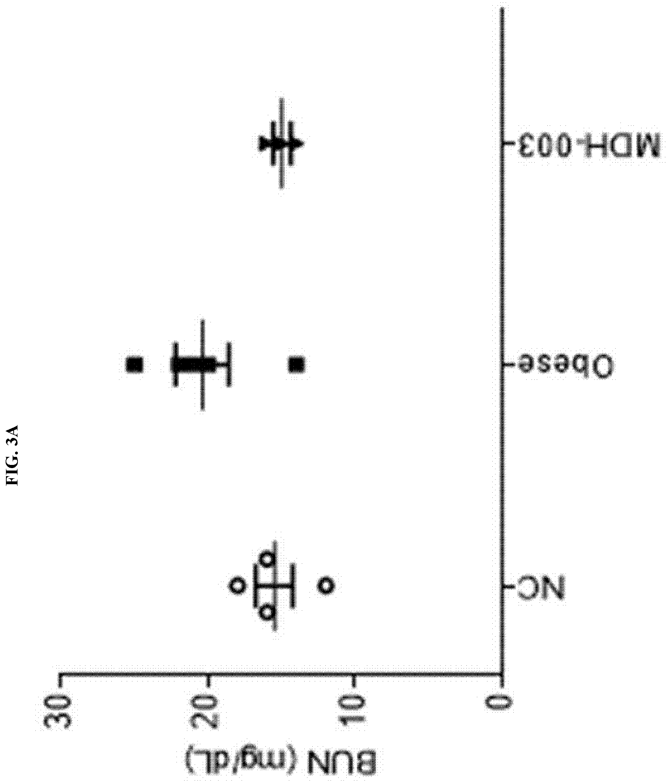

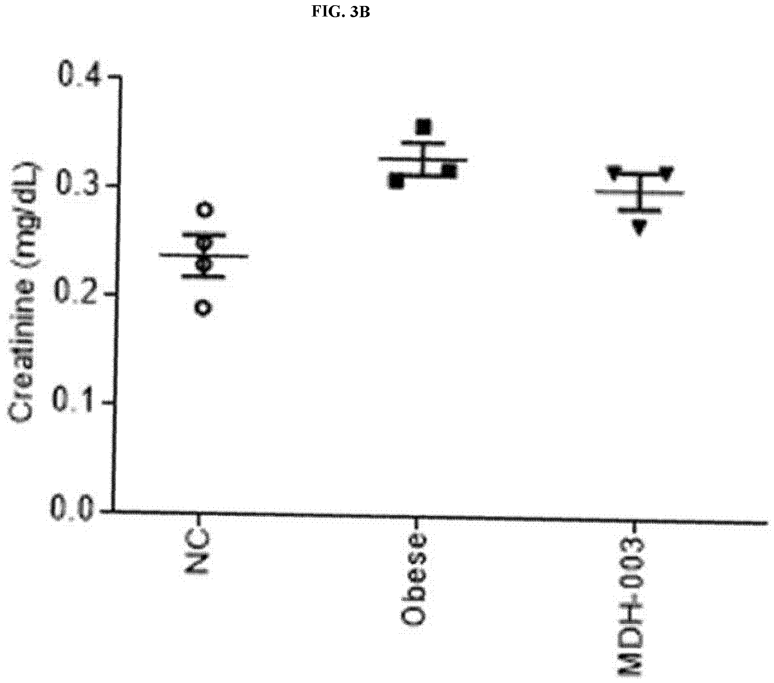

As shown in FIGS. 3A and 3B, the BUN and Cr levels increased in the mice fed a high-fat diet for 27 weeks, and renal dysfunction, that is, kidney damage, induced by the high-fat diet was recovered from in the group to which the *Lactobacillus rhamnosus-derived* vesicles were administered.

### 2-3. Confirmation of pulmonary function improvement effect

Changes in pulmonary function caused by a high-fat diet were evaluated. Specifically, airway hyperresponsiveness (AHR) was measured by using flexiVent (SCIREQ, Canada). This is an invasive method in which an airway resistance (Rrs) value was measured according to changes in methacholine concentration (0, 5, 10, 25, 50 mg/ml) while the mouse was under anesthesia. The basal control airway hyperresponsiveness of the mice was measured by spraying 0.9% saline solution for 10 seconds before having the mice inhale methacholine, and to determine differences in the airway response to methacholine, measurement was performed by increasing the concentration of methacholine to each mouse using an ultrasonic nebulizer. After administering methacholine of different concentrations, the Rrs value was measured for 3 minutes and the average value was used. The degree of change in airway function was expressed as a percentage of the difference based on the baseline Rrs value obtained after the inhalation of the 0.9% saline solution.

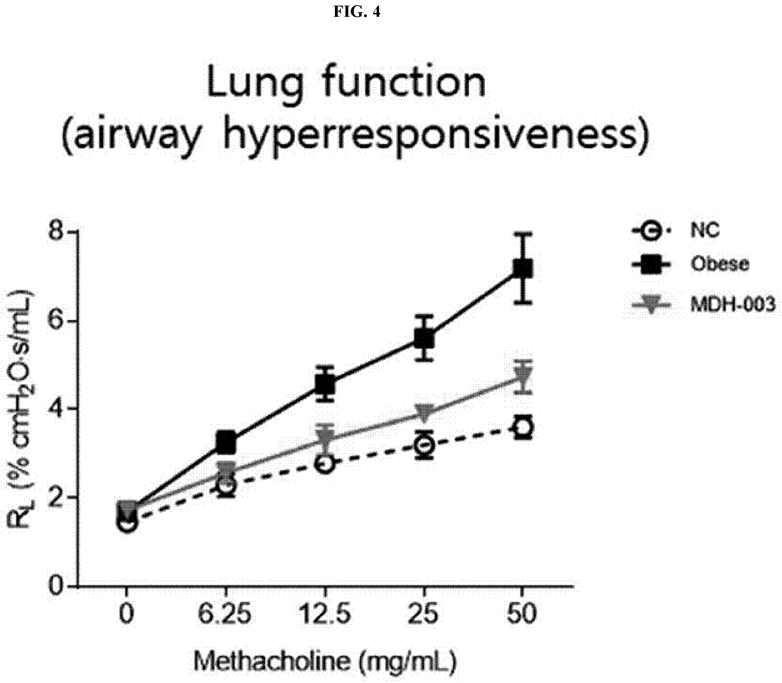

As shown in FIG. 4, airway hyperresponsiveness was increased in the obese group compared to the negative control group, and this increased airway hyperresponsiveness was confirmed to have been improved in the group to which the *Lactobacillus rhamnosus-derived* vesicles were administered.

From the results described above, it can be seen that the *Lactobacillus rhamnosus-derived* vesicles can efficiently treat renal dysfunction and pulmonary dysfunction caused by the acceleration of cellular aging due to overnutrition.

### Example 3. Immune function regulation effect of Lactobacillus rhamnosus-derived vesicles in mice with cellular aging-related disease induced by overnutrition

Immunity may be divided into innate immunity and adaptive immunity, and a disease occurs when immune function is abnormal in response to an external or internal factor. In particular, T cells, which are important in acquired immune responses, induce immune diseases by secreting cytokines. In particular, Th17 cells mainly secrete IL-17, IL-6, etc., thereby inducing inflammatory diseases characterized by neutrophilic inflammation, and Th2 cells secrete IL-4, IL-5, etc., thereby inducing inflammatory diseases characterized by eosinophilic inflammation.

Accordingly, the therapeutic effect of *Lactobacillus rhamnosus-derived* vesicles on hypersensitivity reactions caused by Th17 and Th2 cells was evaluated. Specifically, *Lactobacillus rhamnosus-derived* vesicle were orally administered to mice in which overnutrition was induced by a high-fat diet as in the method of Example 2, and Th17 and Th2-related cytokine (IL-17, IL-4, and IL-5) levels in airway lavage fluid were measured by using an enzyme-linked immunosorbent assay (ELISA, R&D Systems) method. For ELISA analysis, the capture antibody was diluted in PBS, and the resulting solution was dispensed in a volume of 50 µl each to a 96-well polystyrene plate according to the working concentration, and allowed to react overnight at 4°C. After washing twice with 100 µl of a PBST (PBS containing 0.05% Tween-20) solution, 100 µl of an RD (PBST containing 1% BSA) solution was dispensed to perform blocking for 1 hour at room temperature. After washing twice with 100 µl of PBST again, a sample and a standard were dispensed at 50 µl each according to the concentration and allowed to react at room temperature for 2 hours. After washing twice with 100 µl of PBST again, the detection antibody was diluted in RD, dispensed at 50 µl each according to the working concentration and allowed to react at room temperature for 2 hours. After washing twice with 100 µl of PBST again, streptavidin-HRP was diluted 1/200 in RD, dispensed in a volume of 50 µl each, and allowed to react at room temperature for 30 minutes. Finally, after washing three times with 100 µl of PBST, 50 µl of a 1:1 mixture of a TMB substrate and 0.04% hydrogen peroxide was dispensed, and then and then a sufficient amount of time was allowed to pass for color development. When color development progressed for 5 to 20 minutes, 50 µl of a 1 M sulfuric acid solution was dispensed to stop the reaction, and absorbance was measured at 450 nm using a Synergy^{™} HT multi-detection microplate reader (BioTek, USA).

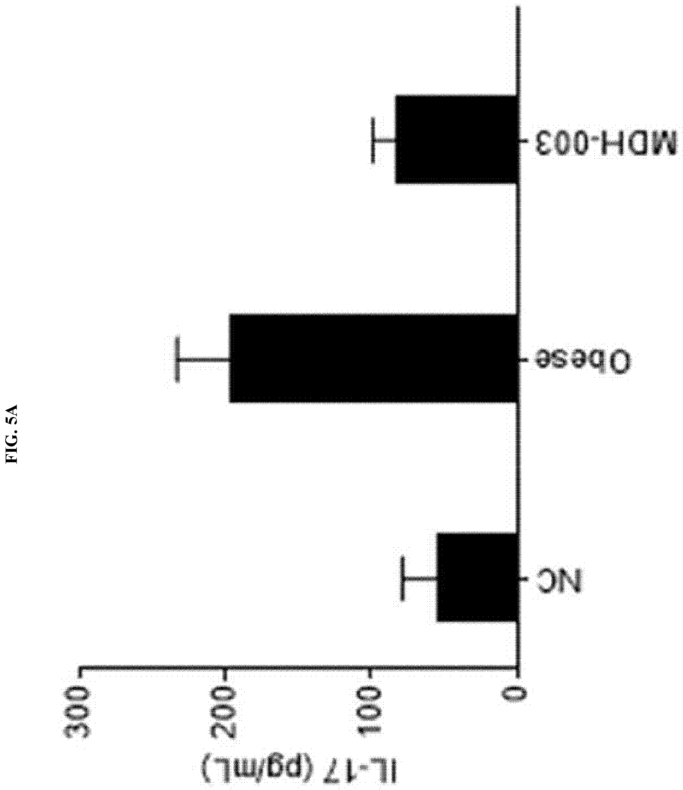

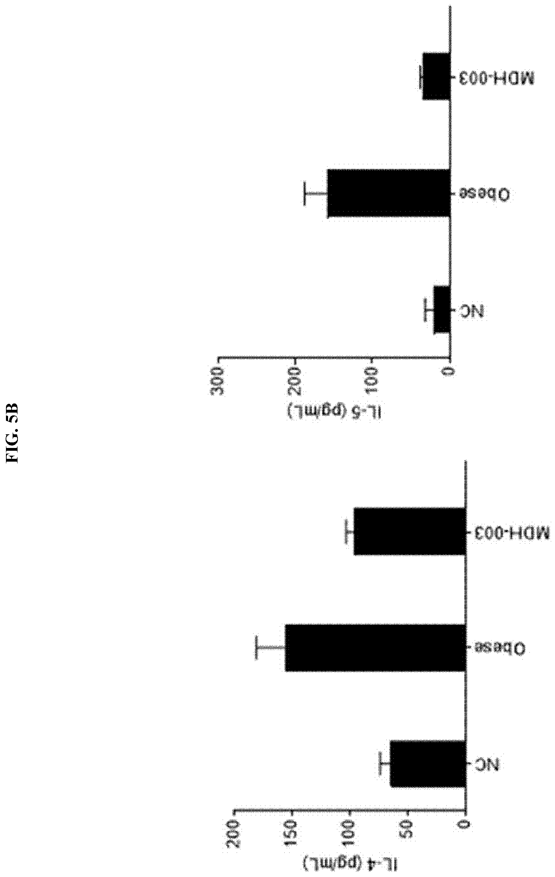

As a result, as shown in FIGS. 5A and 5B, IL-17, which is a Th17 cytokine, and IL-4 and IL-5, which are Th2 cytokines, in the airway lavage fluid were significantly increased in the obese group compared to the mice in a normal nutritional state. On the other hand, in the group to which *Lactobacillus rhamnosus-derived* vesicles were administered, the levels of Th17 and Th2 cytokines in the airway lavage fluid were reduced to normal levels.

Through the results described above, it can be seen that immune dysfunction induced by overnutrition is efficiently inhibited and improved by *Lactobacillus rhamnosus-derived* vesicles.

### Example 4. Therapeutic effect of Lactobacillus rhamnosus-derived vesicles on maintaining cellular homeostasis against energy metabolism stress

Cellular aging is defined as the loss of cell division ability due to repetitive physical, chemical, biological, and mental stress, and together with aging of cells due to repetitive stress, cellular aging and aging-related diseases occur as cell regeneration ability decreases. In particular, cells activate the AMPK protein as an intracellular signaling pathway in metabolic stress situations where ATP production is low, thereby inducing metabolic homeostasis of cells and suppressing cellular aging and apoptosis.

Therefore, an experiment was performed in the following manner to evaluate the effect of *Lactobacillus rhamnosus-derived* vesicles (MDH-003) on cellular aging through AMPK activation within cells. That is, to evaluate AMPK activity according to the concentration *of Lactobacillus rhamnosus-derived* vesicles in muscle cells, cells were treated with *Lactobacillus rhamnosus-derived* vesicles at concentrations of 0, 0.1, 1, and 10 µg/ml for 1 hour. As a control group, metformin, which suppresses aging, was used at a concentration of 10 µg/ml. After treating the cells with the drug, the difference in the amount of pAMPK, which is an important indicator in AMPK signaling, was measured by Western blotting.

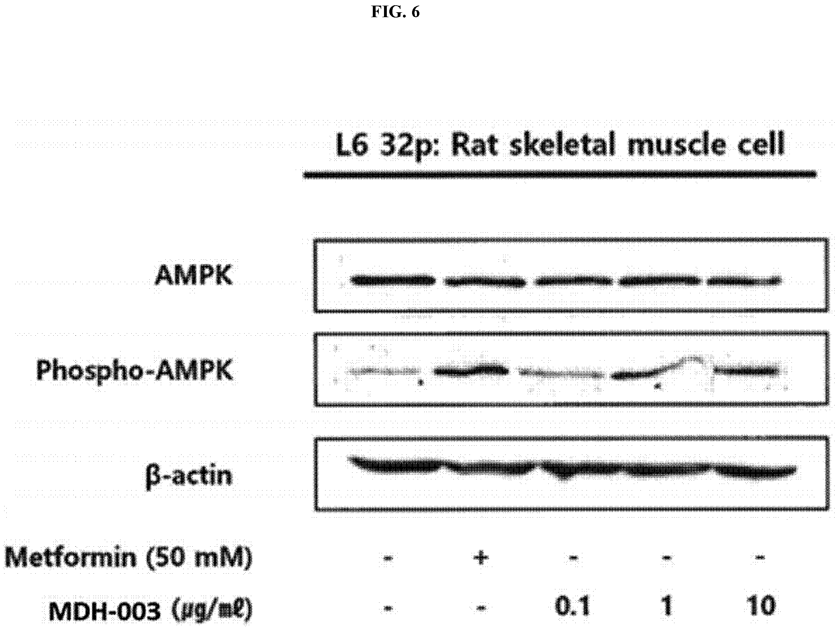

As a result, as shown in FIG. 6, phosphorylation of AMPK (pAMPK) was enhanced similarly to metformin in the case where *Lactobacillus rhamnosus-derived* vesicles were treated, and this effect increased depending on the concentration of the vesicles of the present invention.

Through the results described above, it can be seen that *Lactobacillus rhamnosus-derived* vesicles increase cellular homeostasis against metabolic stress by activating AMPK signaling, thereby enhancing cell viability and preventing aging.

### Example 5. Inflammation inhibitory effect of Lactobacillus rhamnosus-derived vesicles in a mouse model of immune disease induced by viral causative agent poly(I:C)

Poly(I:C) is an artificially synthesized double-structured ribonucleotide polymer that is structurally similar to dsRNA present in some viruses and activates various inflammatory signaling pathways to cause viral infectious diseases.

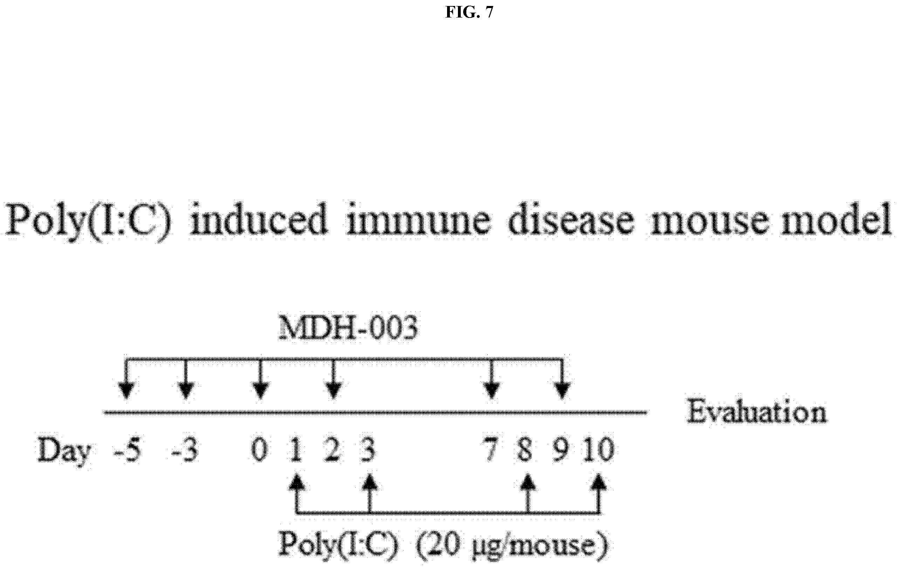

Accordingly, an experiment was performed in the following manner to evaluate the therapeutic effect of *Lactobacillus rhamnosus-derived* vesicles in an immune disease model induced by a viral causative agent. That is, as shown in FIG. 7, an immune disease mouse model was created by intranasally administering poly(I:C), a viral causative factor, to C57BL/6 6-week-old male mice every other day for 2 weeks. *Lactobacillus rhamnosus-derived* vesicles were orally administered every other day for 2 weeks, starting at 6 days before poly(I:C) administration. As a control drug, dexamethasone was intraperitoneally administered at a dose of 2 mg/kg.

### 5-1. Confirmation of increased inflammatory cell reduction effect

After administration, airway lavage fluid from the mouse was obtained, and the number of inflammatory cells was evaluated.

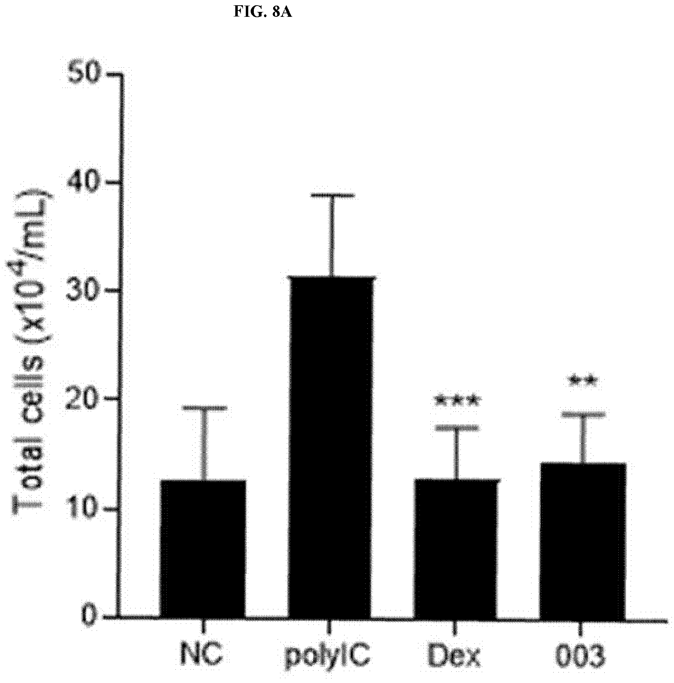

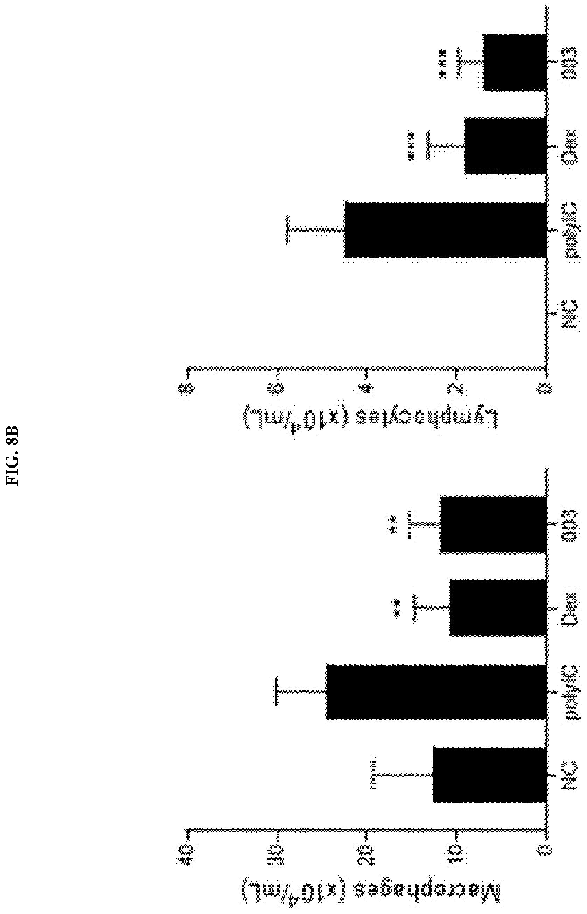

As a result, as shown in FIGS. 8A and 8B, changes in the number of inflammatory cells in the airway lavage fluid were observed. As a result, in the case where poly(I:C) was administered, the number of inflammatory cells in the airway lavage fluid increased, especially the number of macrophages and lymphocytes increased. Meanwhile, the number of inflammatory cells in the airway lavage fluid, which was increased by poly(I:C), was significantly inhibited by *Lactobacillus rhamnosus-derived* vesicles, similar to dexamethasone (Dex) treatment.

### 5-2. Confirmation of pulmonary tissue damage alleviating effect

Changes in pulmonary tissue were evaluated by hematoxylin and eosin (H&E) staining.

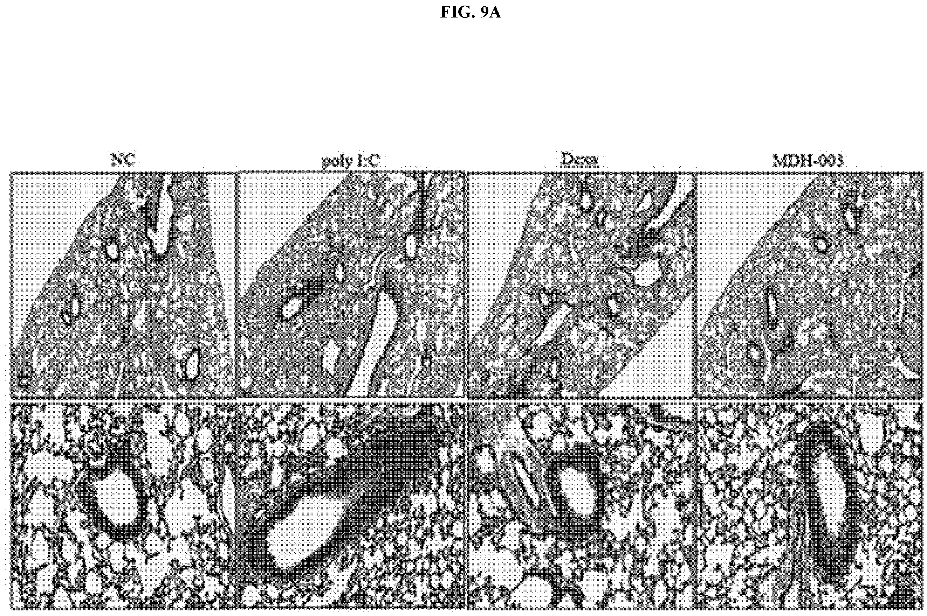

As shown in FIG. 9A, small-sized alveoli were uniformly observed in the pulmonary tissue of the normal group, while in the poly(I:C) administered group, it was observed that many cells were clustered around the alveoli. In addition, the shape of the alveoli was maintained relatively uniform in the group treated with dexamethasone and the group to which *Lactobacillus rhamnosus-derived* vesicles were administered.

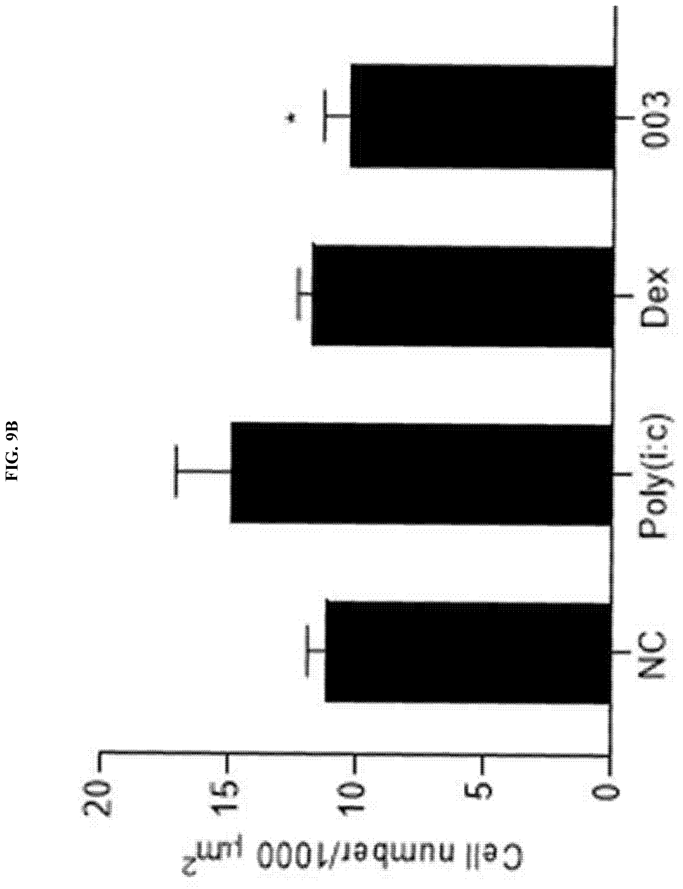

Additionally, as shown in FIG. 9B, it was confirmed that infiltration of immune cells around the airway decreased.

### 5-3. Confirmation of systemic inflammatory response inhibiting effect

A systemic inflammatory response caused by a viral causative agent was evaluated through a blood high-sensitivity C-reactive protein (hsCRP) level. CRP is an acute phase reactive protein that has been used as a marker for systemic inflammatory responses, and hsCRP is not a simple marker of cardiovascular disease but an independent risk factor. The hsCRP test can more accurately detect lower concentrations of the protein than the standard CRP test. It is accepted that hsCRP plays an important role in the occurrence of arteriosclerosis and complications caused thereby through actions such as acceleration of dysfunction of vascular endothelial cells and enhancement of blood clot formation. In this Example, the hsCRP concentration was measured using the immunonephelometric assay method.

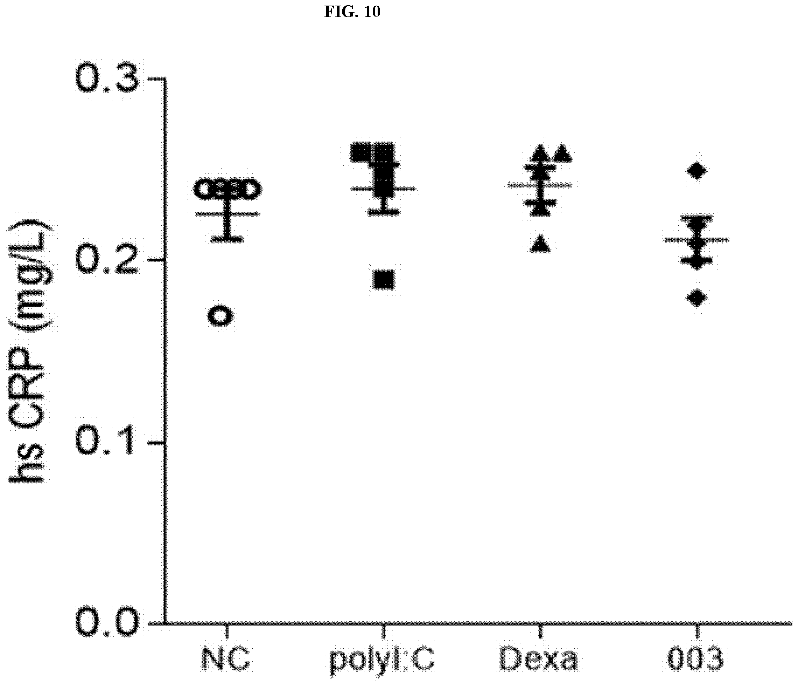

As shown in FIG. 10, the hsCRP level was decreased by the *Lactobacillus rhamnosus-derived* vesicles.

Through the results described above, it can be seen that systemic inflammatory responses and pulmonary diseases induced by poly(I:C) can be effectively treated by *Lactobacillus rhamnosus-derived* vesicles.

### Example 6. Immunomodulatory effect of Lactobacillus rhamnosus-derived vesicles in a mouse model of immune disease induced by viral causative agent poly(I:C)

### 6-1. Confirmation of secretion of inflammatory cytokines in airway lavage fluid

TNF-α and IL-6 secreted by inflammatory cells are representative inflammatory mediators that induce inflammatory diseases caused by pathogenic causative factors. In this Example, an experiment was performed in the following manner to evaluate the immunomodulatory effect *of Lactobacillus rhamnosus-derived* vesicles on immune dysfunction caused by viral causative factors. That is, in the immune disease mouse model described in Example 5, inflammatory cytokines TNF-α and IL-6 in airway lavage fluid were measured by an ELISA method. The ELISA method described above was used.

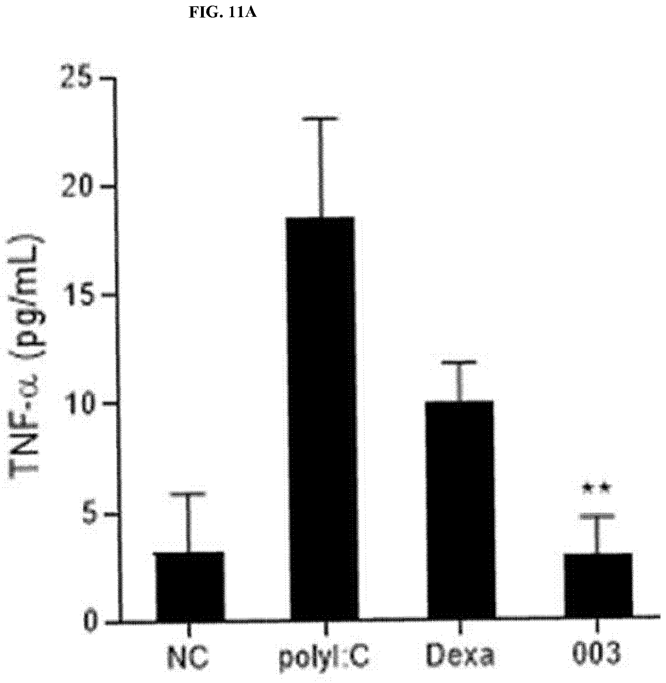

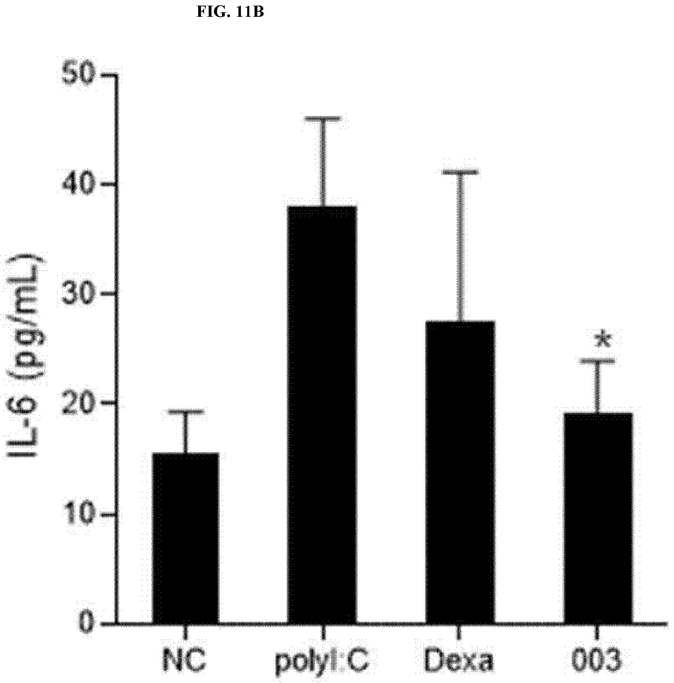

As a result, as shown in FIGS. 11A and 11B, the concentrations of TNF-α and IL-6 in the airway lavage fluid increased in the poly(I:C) administration group compared to the negative control (NC) group. On the other hand, the secretion of TNF-α and IL-6 increased by poly(I:C) was significantly reduced in the group to which *Lactobacillus rhamnosus-derived* vesicles were administered to the level of the negative control group, and the inhibitory effect was better compared to the dexamethasone administration group (Dexa).

### 6-2. Confirmation of inflammatory cytokine secretion from splenic T cells

The therapeutic effect of *Lactobacillus rhamnosus-derived* vesicles on systemic immune dysfunction caused by poly(I:C) was evaluated by evaluating the cytokine secretion pattern in splenic T cells.

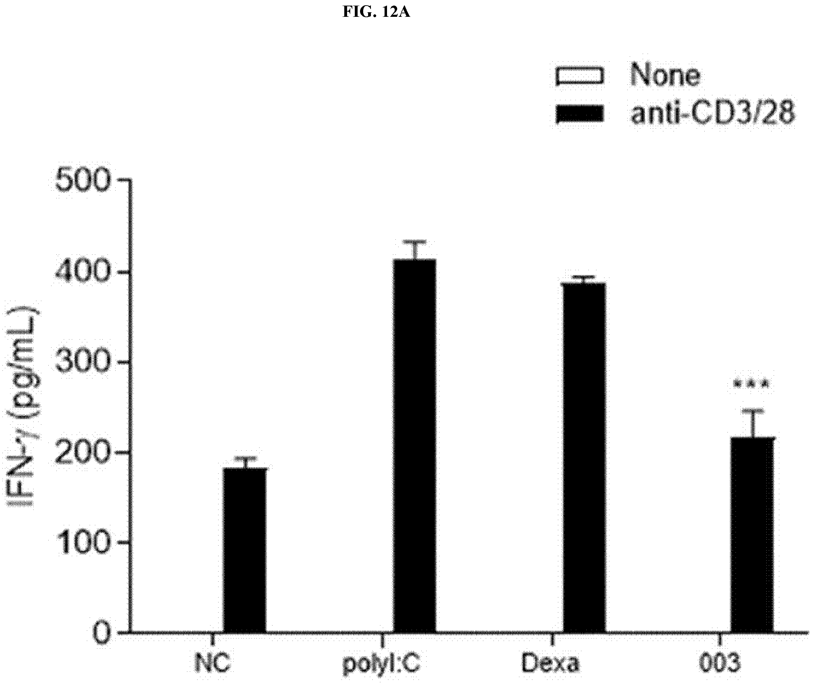

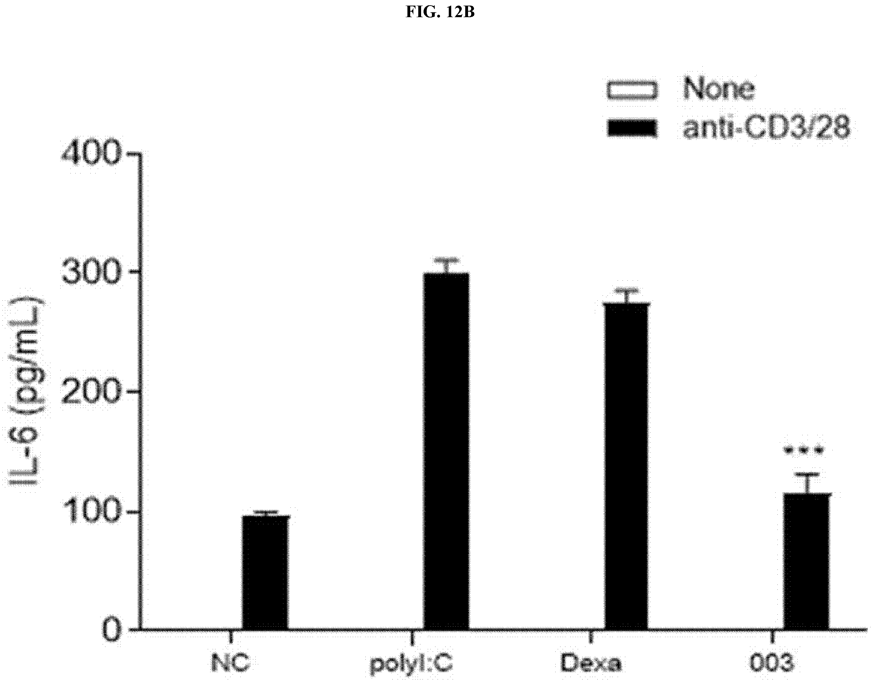

As shown in FIGS. 12A and 12B, in the poly(I:C) administration group, the secretion of Th1 cytokine IFN-γ and Th17 cytokine IL-6 was increased compared to the negative control group. On the other hand, the increased levels of IFN-γ and IL-6 were significantly inhibited by the *Lactobacillus rhamnosus-derived* vesicles. Meanwhile, dexamethasone (Dexa) was ineffective.

Through the results described above, it can be seen that *Lactobacillus rhamnosus-derived* vesicles can efficiently inhibit immune dysfunction caused by viral causative factors, and that there is a superior therapeutic effect than that of dexamethasone, a conventional immunosuppressant.

### Example 7. Confirmation of molecular biological action mechanism of Lactobacillus rhamnosus-derived vesicles on the regulation of immune dysfunction caused by viral causative factor poly(I:C)

Nuclear factor-κB (NF-κB), which is a nuclear transcription factor, is an inflammation regulator that induces the transcription of inducible nitric oxide synthase (iNOS) and increases the secretion of various proinflammatory cytokines.

Accordingly, to evaluate the effect of *Lactobacillus rhamnosus-derived* vesicles on inflammatory responses through NF-κB activation, an experiment was performed by the Western blotting method using mouse lung tissue.

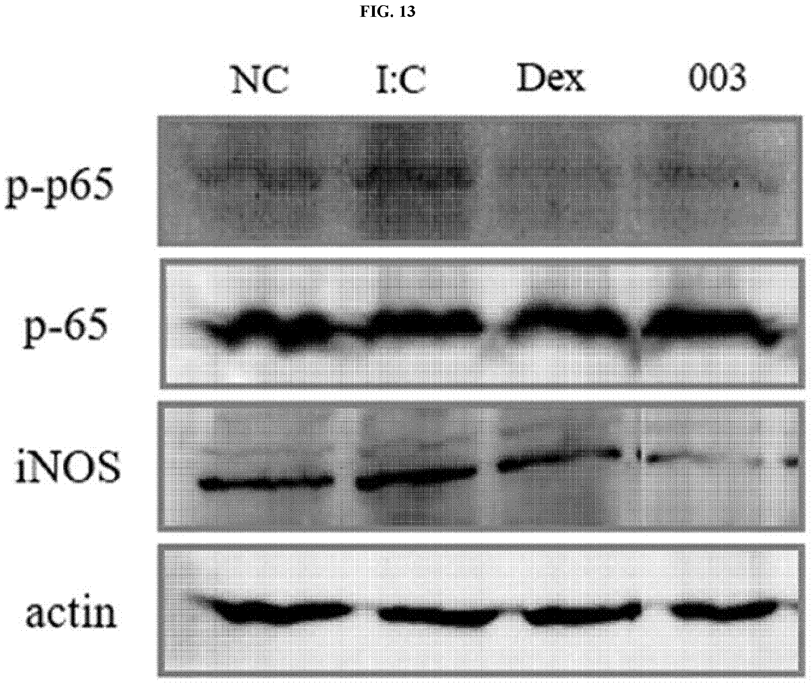

As shown in FIG. 13, the phosphorylation of NF-κB (p-NF-κB, p-p65) and iNOS expression increased in the case where poly(I:C) was administered. In addition, the phosphorylation of NF-κB (p-NF-κB, p-p65) increased by poly(I:C) was inhibited by dexamethasone (Dex) and the *Lactobacillus rhamnosus-derived* vesicles. In addition, in the case where *Lactobacillus rhamnosus-derived* vesicles were administered, iNOS expression increased by poly(I:C) was inhibited more efficiently compared to dexamethasone.

Through the results described above, it can be seen that *Lactobacillus rhamnosus-derived* vesicles efficiently inhibit the expression of an NF-κB signal, which is the key to immune dysfunction caused by pathogenic causative factors, and the downstream signal thereof, iNOS, to control immune hypersensitivity reactions caused by viral causative agents.

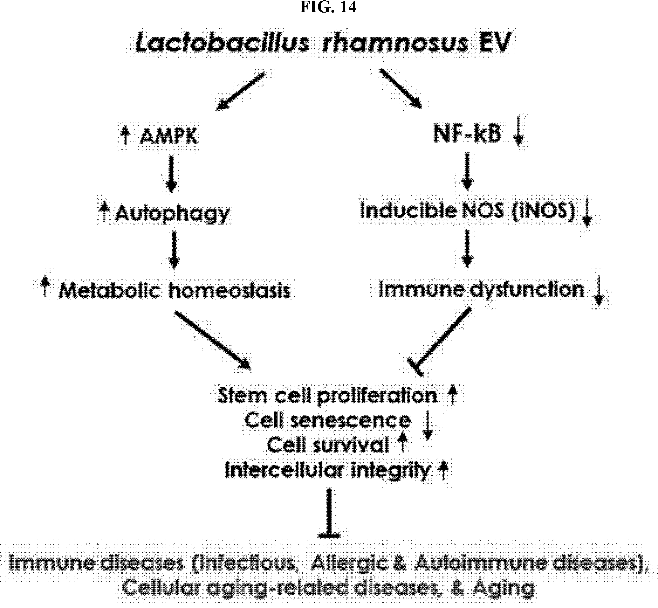

Through the results described above, it was confirmed that the *Lactobacillus rhamnosus-derived* vesicle of the present invention can not only suppress cellular aging and apoptosis by increasing of cellular homeostasis through AMPK signaling, but also suppress immune dysfunction caused by biological causative factors by inhibiting NF-κB signaling to efficiently treat immune diseases and cellular aging-related diseases (see FIG. 14). Therefore, it is expected that the *Lactobacillus rhamnosus-derived* vesicles of the present invention may be used for alleviating, preventing, or treating diseases related to immune dysfunction and cellular aging.

The description of the present invention presented above is for illustrative purposes, and it should be understood that one of ordinary skill in the art to which the present invention can easily make modifications into other specific forms without changing the technical ideas or essential features of the present invention. Therefore, the embodiments described above should be understood in all respects as illustrative and not restrictive.

### [Industrial Applicability]

The *Lactobacillus rhamnosus-derived* vesicle according to the present invention can suppress weight gain and alleviate renal and pulmonary functions, and it activated AMPK, which a key signal that induces cellular homeostasis, inhibited systemic inflammatory responses, and significantly inhibited pulmonary inflammation caused by immune hypersensitivity reactions. Therefore, it has industrial applicability because it can be effectively used in the development of a drug or health functional food for preventing, alleviating, or treating immune diseases including viral infectious diseases, allergic immune diseases, and autoimmune diseases, and cellular aging-related diseases.

## Claims

1. A pharmaceutical composition for preventing or treating one or more selected from the group consisting of immune diseases and cellular aging-related diseases, comprising vesicles derived from *Lactobacillus rhamnosus* as an active ingredient.

2. The pharmaceutical composition of claim 1, wherein the immune diseases is one or more selected from the group consisting of viral infectious diseases, allergic immune diseases, and autoimmune diseases.

3. The pharmaceutical composition of claim 2, wherein the viral infectious disease is one or more selected from the group consisting of viral pneumonia, viral hepatitis, viral carditis, viral enteritis, viral nephritis, viral thromboembolism, shingles, and Meniere's disease

4. The pharmaceutical composition of claim 2, wherein the autoimmune disease is one or more selected from the group consisting of systemic lupus erythematosus, rheumatoid arthritis, Sjogren's syndrome, Behcet's disease, ankylosing spondylitis, and multiple sclerosis.

5. The pharmaceutical composition of claim 2, wherein the allergic immune disease is one or more selected from the group consisting of atopic dermatitis, psoriasis, allergic conjunctivitis, allergic rhinitis, allergic asthma, hypersensitivity pneumonitis, food allergies, celiac disease, irritable bowel syndrome, and anaphylaxis.

6. The pharmaceutical composition of claim 1, wherein the cellular aging-related disease is one or more selected from the group consisting of:
one or more cardiovascular diseases selected from the group consisting of angina pectoris, myocardial infarction, cardiomyopathy, thromboembolism, and stroke;
one or more hepato-biliary-pancreatic diseases selected from the group consisting of alcoholic steatohepatitis, cirrhosis, chronic cholangitis, chronic cholecystitis, and chronic pancreatitis;
one or more musculoskeletal diseases selected from the group consisting of gout, osteoarthritis, and osteoporosis; and
one or more neurological diseases selected from the group consisting of Alzheimer's disease, Parkinson's disease, and macular degeneration.

7. The pharmaceutical composition of claim 1, wherein the vesicles have an average diameter of 10 to 1000 nm

8. The pharmaceutical composition of claim 1, wherein the vesicles are isolated from the culture medium of *Lactobacillus rhamnosus.*

9. The pharmaceutical composition of claim 1, wherein the vesicles are isolated from food manufactured by adding *Lactobacillus rhamnosus.*

10. The pharmaceutical composition of claim 1, wherein the vesicles are naturally secreted or artificially produced from *Lactobacillus rhamnosus.*

11. A food composition for preventing or alleviatiing one or more selected from the group consisting of immune diseases and cellular aging-related diseases, comprising vesicles derived from *Lactobacillus rhamnosus* as an active ingredient.

12. The food composition of claim 11, wherein the immune diseases is one or more selected from the group consisting of viral infectious diseases, allergic immune diseases, and autoimmune diseases.

13. The food composition of claim 11, wherein the vesicles are isolated from the culture medium of *Lactobacillus rhamnosus.*

14. The food composition of claim 11, wherein the vesicles are isolated from food manufactured by adding *Lactobacillus rhamnosus.*

15. The food composition of claim 11, wherein the vesicles are naturally secreted or artificially produced from *Lactobacillus rhamnosus.*

16. A pharmaceutical composition for preventing or treating an aging, comprising vesicles derived from *Lactobacillus rhamnosus* as an active ingredient.

17. A food composition for preventing or alleviating an aging, comprising vesicles derived from *Lactobacillus rhamnosus* as an active ingredient.

18. A cosmetic composition for preventing or alleviating an aging, comprising vesicles derived from *Lactobacillus rhamnosus* as an active ingredient.

19. A method for preventing or treating one or more selected from the group consisting of immune diseases and cellular aging-related diseases, the method comprising administering a composition comprising vesicles derived from *Lactobacillus rhamnosus* as an active ingredient to a subject in need thereof.

20. A use of a composition comprising vesicles derived from *Lactobacillus rhamnosus* as an active ingredient for preventing or treating one or more selected from the group consisting of immune diseases and cellular aging-related diseases.

21. A use of vesicles derived from *Lactobacillus rhamnosus* for manufacturing a therapeutic agent for treating one or more selected from the group consisting of immune diseases and cellular aging-related diseases.

22. A method for preventing or treating an aging, the method comprising administering a composition comprising vesicles derived from *Lactobacillus rhamnosus* as an active ingredient to a subject in need thereof.

23. A use of a composition comprising vesicles derived from *Lactobacillus rhamnosus* as an active ingredient for preventing or treating an aging.

24. A use of vesicles derived from *Lactobacillus rhamnosus* for manufacturing a therapeutic agent for treating an aging.
